# EUROPEAN PATENT SPECIFICATION

(11) **EP 1 463 531 B1**
(45) Date of publication and mention of the grant of the patent: **28.09.2011**
(21) Application number: 02804786.8
(22) Date of filing: 11.12.2002
(51) Int. Cl.: A61K 48/00, A61K 39/395, G01N 33/53, A61P 27/02

(54) **METHODS FOR INHIBITING OCULAR PROCESSES**
VERFAHREN ZUR HEMMUNG OKULARER VORGÄNGE
PROCEDE POUR INHIBER DES PROCESSUS OCULAIRES

(30) Priority: 11.12.2001 US 339547 P
(43) Date of publication of application: 06.10.2004
(73) Proprietor: FIBROGEN, INC., San Francisco, CA 94158 (US); UNIVERSITY OF SOUTHERN CALIFORNIA, Los Angeles, CA 90089 (US)
(72) Inventor: HINTON, David, R., Venice, CA 90291 (US); HE, Shikun, Temple City, CA 91780 (US); OLIVER, Noelynn, A., Los Altos, CA 94022 (US)
(74) Representative: Goodfellow, Hugh Robin
(86) International application number: PCT/US2002/039777
(87) International publication number: WO 2003/049773

(56) References cited:
- WO-A-00/35936
- WO-A-01/15729
- WO-A-02/07747
- WO-A-99/13910
- WO-A-03/024308
- US-A1- 2003 012 768
- US-B1- 6 455 283
- WUNDERLICH K ET AL: "Expression of connective tissue growth factor (CTGF) mRNA in plaques of human anterior subcapsular cataracts and membranes of posterior capsule opacification." CURRENT EYE RESEARCH. AUG 2000, vol. 21, no. 2, August 2000 (2000-08), pages 627-636, XP009050313 ISSN: 0271-3683
- WATANABE D ET AL: "Expression of connective tissue growth factor in human choroidal neovascular membranes" IOVS, vol. 42, no. 4, 15 March 2001 (2001-03-15), page S228, XP009050332 & ANNUAL MEETING OF THE ASSOCIATION FOR RESEARCH IN VISION AND OPHTHALMOLOGY; FORT LAUDERDALE, FLORIDA, USA; APRIL 29-MAY 04, 2001
- VAN SETTEN G -B ET AL: "Detection of connective tissue growth factor in human aqueous humor." OPHTHALMIC RESEARCH, vol. 34, no. 5, September 2002 (2002-09), pages 306-308, XP009050312 ISSN: 0030-3747
- MEYER PETER ET AL: "Human connective tissue growth factor mRNA expression of epiretinal and subretinal fibrovascular membranes: A report of three cases" OPHTHALMOLOGICA, vol. 216, no. 4, July 2002 (2002-07), pages 284-291, XP009050311 ISSN: 0030-3755
- HISHIKAWA ET AL.: 'Connective tissue growth factor induces apoptosis in human breast cancer cell line MCF-7' THE JOURNAL OF BIOLOGICAL CHEMISTRY vol. 274, no. 52, 24 December 1999, pages 37461 - 37466, XP002966506
- SHIMO ET AL.: 'Inhibition of endogenous expression of connective tissue growth factor by its antisense oligonucleotide and antisense RNA suppresses proliferation and migration of vascular endothelial cells' J. BIOCHEM. vol. 124, 1998, pages 130 - 140, XP009009314
- 'CTGF mediates TGF-beta-induced fibronectin matrix deposition by upregulating active alpha5beta1 integrin in human mesangial cells' J. AM. SOC. NEPHROL. vol. 14, 2003, pages 601 - 610, XP002966505
- VAN DER PIJL J.W. ET AL: 'Effect of danaparoid sodium on hard exudates in diabetic retinopathy' LANCET THE, LANCET LIMITED. LONDON, GB LNKD- DOI:10.1016/S0140-6736(97)07126-2 vol. 350, no. 9093, 13 December 1997, pages 1743 - 1745, XP004265031 ISSN: 0140-6736
- CREE M.J. ET AL: 'The preprocessing of retinal images for the detection of fluorescein leakage.' PHYSICS IN MEDICINE AND BIOLOGY JAN 1999 LNKD- PUBMED:10071890 vol. 44, no. 1, January 1999, pages 293 - 308 ISSN: 0031-9155
- YOUNG R.W.: 'PATHOPHYSIOLOGY OF AGE-RELATED MACULAR DEGENERATION' SURVEY OF OPHTHALMOLOGY vol. 31, no. 5, 1987, pages 291 - 306, XP023266110 ISSN: 0039-6257
- PENFOLD P.L. ET AL: 'Immunological and aetiological aspects of macular degeneration.' PROGRESS IN RETINAL AND EYE RESEARCH MAY 2001 LNKD- PUBMED:11286898 vol. 20, no. 3, May 2001, pages 385 - 414 ISSN: 1350-9462
- KIMURA HIDEYA ET AL: "Drug targeting to choroidal neovascularization", ADVANCED DRUG DELIVERY REVIEWS, vol. 52, no. 1, 31 October 2001 (2001-10-31), pages 79-91, ISSN: 0169-409X
- STONE EDWIN M.: 'Macular degeneration.' ANNUAL REVIEW OF MEDICINE 2007 LNKD- PUBMED:16922634 vol. 58, 2007, pages 477 - 490 ISSN: 0066-4219
- JFFYTCHE T.J. ET AL: 'Indications for fluorescein angiography in disease of the ocular fundus: A review' JOURNAL OF THE ROYAL SOCIETY OF MEDICINE 1980 GB vol. 73, no. 5, 1980, pages 362 - 365 ISSN: 0141-0768

## Description

### FIELD OF THE INVENTION

The present disclosure relates generally to methods for inhibiting or preventing ocular processes, including extracellular matrix production and/or deposition, neovascularization, inflammation, cell proliferation, cell migration, etc., in the eye, and specifically to the agents of claim 1 and uses of claim 14.

### BACKGROUND OF THE INVENTION

The eye is a complex organ, and possesses multiple functional layers containing various types of structures, including highly specialized cell types. The retina is the light-sensitive layer lining the back of the eye. The macula is the portion of the retina lying on the optical axis of the eye., The macula provides detailed vision and distinguishes fine details at the center of the field of vision. In a healthy eye, the outer retina is nourished and maintained by an adjoining layer called the retinal pigment epithelium, a monolayer of retinal pigment epithelial (RPE) cells located beneath the retina. Behind the retinal pigment epithelium is the choroid, a highly vascular region containing blood vessels that transport nourishment to and carry waste material away from the retina. Other important structures of the eye include the lens, iris, cornea, trabecular meshwork, and vitreous.

An ocular disorder is any alteration in normal physiology and function of the eye. Ocular disorders can result from a wide range of acute or chronic conditions and events, including injury, insult, or trauma; disease; surgery; etc. Ocular disorders can lead to reduced optical clarity, vision loss, and blindness, seriously compromising quality of life.

The vast majority of ocular disorders are associated with certain physiological processes. Cell proliferation, cell migration, inflammation, neovascularization, and fibrosis (excess production and deposition of extracellular matrix components) develop in response to or in association with, or lead to, changes in ocular morphology and function, and any number of ocular diseases and conditions. Any wound healing response in the eye often includes one or more of these processes.

Examples of ocular disorders, and the ocular processes common to them, include the following:

### Proliferative vitreoretinopathy

Proliferative vitreoretinopathy (PVR) is an ocular disorder resulting from any ocular injury or trauma, including, for example, perforating injury, rhegmatogenous retinal detachment, penetrating injury, intraocular foreign body, contusion, etc. (Cardillo et al. (1997) Ophthalmology 104:1166-1173.) PVR is the most common cause of treatment failure in rhegmatogenous retinal detachment, associated with retinal distortion, breaks, and recurrent detachment. (Girard et al. (1994) Retina 14:417-424.) PVR is associated with cellular proliferation and formation, growth, and contraction of cellular and fibrotic membranes within the vitreous cavity (vitreous membranes) and on both the inner and outer surfaces of the retina (epiretinal membranes). RPE cells proliferation and migration is a major feature of this disorder.

Epiretinal and vitreous membranes contain predominantly RPE cells, but can also contain fibroblasts, glial cells (*e.g*., Müller cells, astrocytes, microglia, perivascular glia, etc.), and inflammatory cells (*e.g*., macrophages, lymphocytes, etc.). In addition to cellular components, epiretinal and vitreous membranes, such as the membranes associated with PVR, contain extracellular matrix components, such as collagens (*e.g*., collagen types I, II, and IV) and fibronectin, and can also contain heparan sulphate, laminin, vitronectin, thrombospondin, etc. The epiretinal and vitreous membranes associated with PVR become progressively paucicellular and fibrotic over time. (Hiscott et al. (1985) Br J Ophthalmol 11:810-823.) PVR and other conditions associated with development of or alterations in ocular membranes can occur as complications of a number of diseases affecting the eye, such as, for example, diabetes.

### Macular degeneration

Macular degeneration, including age-related macular degeneration, is a leading cause of decreased visual acuity and impairment of reading and fine close-up vision. Generally, macular degeneration is characterized by degeneration of the central portion of the retina, known as the macula, resulting in loss of central vision in the affected eye. Degeneration of the macula originates, at least in part, in the retinal pigment epithelium. Macular degeneration is associated with abnormal deposition of extracellular matrix in the basement membrane (Bruch's membrane) found between the retinal pigment epithelium and the vascular choroid. The deposits of extracellular matrix in macular degeneration are called drusen. Two major forms of macular degeneration have been characterized: dry form of macular degeneration, also known as non-exudative, involutional, atrophic macular degeneration or geographic atrophy; and wet form of macular degeneration, also known as exudative, neovascular, or disciform macular degeneration.

### Choroidal neovascularization

Choroidal neovascularization (CNV) is a serious condition, often associated with macular degeneration involving increased choroidal endothelial cell (CEC) adhesion, migration, and proliferation; extracellular matrix overproduction; and development of a fibrovascular subretinal membrane. (D'Amore (1994) IOVS 35:3974-3979; Hinton et al. (1998) Arch Ophthahnol 116:203-209.) Evidence suggests that the retinal pigment epithelium plays a key role in the development of CNV, and that RPE cell dedifferentiation, proliferation, migration, and production of angiogenic factors modulate CNV development. (Grossniklaus et al. (1992) Am J Ophthamol 114:464-472; Das et al. (1992) Ophthamology 99:1368-1376.) While CNV most commonly occurs in macular degeneration, it has been identified as a complication of over forty other ocular disorders.

### Wound healing

Wound healing in response to acute or chronic injury, trauma, disease, etc., can become prolonged or dysregulated, thus leading to more severe ocular tissue damage and loss of vision. The wound healing cascade can include, *e.g*., initiation of an inflammatory response, recruitment and activation of inflammatory cells, release of growth factors and cytokines, activation, proliferation, migration, and differentiation of various ocular cells, increased capillary permeability, alterations in basement membrane matrix composition, and increased secretion and deposition of extracellular matrix by various ocular cells, fibrosis, neovascularization, tissue remodeling, and tissue repair. (Cordeiro et al. (1999) Br J Ophthalmol 83:1219-1224.)

As the above discussion illustrates, a wide range of ocular disorders are interrelated and encompass common processes. The conditions described above, and other, including, for example, diabetic retinopathy and glaucoma, exemplify the association of serious ocular conditions with these common ocular processes, *e.g*., ocular neovascularization and ocular scar formation (*i.e.*, ocular fibrosis).

Despite the involvement of common physiological processes such as those described above in the vast majority of ocular disorders, current therapies fail to address these universal mechanisms of ocular disease, and can thus be limited in application. Specifically, no current therapies are directed to treatment of processes such as ocular fibrosis, scar formation, and ocular membrane formation, *e.g.,* epiretinal membrane formation, common to a large number of ocular disorders.

Available treatments for diabetic retinopathy, for example, have been somewhat efficacious in addressing regression of new blood vessel formation and prevention of vitreous hemorrhage and tractional retinal detachments. However, this therapeutic , approach does not address, and thus fails to prevent or minimize, the development and progression of various processes (*e.g*., cell proliferation, cell migration, ocular fibrosis, etc.) that, unchecked, can lead to vision impaired in varying degrees of severity, and even blindness. Current treatments PVR include vitrectomy, involving the removal of vitreous and membranes from the surface of retina; however, ocular inflammation is induced as a result of such treatment, leading to reactivation of pathological processes associated with PVR. In treatment for macular degeneration associated with CNV, photodynamic/laser photocoagulation therapy for occlusion of activated choroidal vessels does not address associated ocular fibrosis, and, in fact, can even induce a wound healing response, that can lead to further complications. Many other current treatments for ocular disorders often apply mechanical means, such as surgery, including laser surgery, to remove or ablate membranes or abnormal ocular tissue, rather than being directed to growth factors and other pathological molecules which mediate ocular processes.

In summary, no current treatments are successfully directed to prevent or inhibit further development and progression in ocular diseases with processes including, *e.g.,* neovascularization, fibrosis, cell proliferation, cell migration, inflammation, etc. There is thus a need for a therapeutic approach that can be broadly applied to treatment of a number of ocular disorders, regardless of origin or development. Further, there is a need for a therapeutic approach that addresses ocular processes universal to the development and progression of ocular disease. In particular, there is a need for therapies that target the fibrotic process and ocular scarring extremely prevalent in macular degeneration, diabetic retinopathy, cataract, and corneal fibrosis, including corneal scarring or hazing, and glaucoma.

Until the present diclosure, the role of connective tissue growth factor (CTGF) in various ocular processes and associated disorders has remained unclear. CTGF expression in cultured vascular retinal endothelial cells, lens epithelial cells, and rabbit corneal fibroblasts has been reported. (See, *e.g*., Suzuma et al. (2000) J Biol Chem 275:40725-40731; Lee and Joo (1999) Invest Ophthalmol Vis Sci 40:2025-2032; Park et al. (2001) Biochem Biophys Res Commun 284:966-971; Folger et al. (2001) Invest Ophthalmol Vis Sci 42:2534-2541.) CTGF mRNA expression was observed in corneal scar tissue and retrocomeal membranes, in human anterior subcapsular cataracts and membranes of posterior capsule opacification, and in epiretinal and subretinal fibrovascular membranes. (Wunderlich et al. (2000) Ophthalmologica 214:341-346; Wunderlich et al. (2000) Curr Eye Res 21:627-636; Meyer et al. (2002) Ophthalmologica 216:284-291.) These preliminary observations suggest a need in the art exists for a more complete understanding and characterization of the role of CTGF in ocular disorders.
WO 00/35936, WO 99/13910 and WO 01/45729 disclose the use of anti-CTGF antibodies and anti-sense nucleotides targeting this molecule for the treatment of glaucoma and diabetic retinopathy

In summary, there is a need for therapeutic approaches that can be applied to the prevention or treatment of ocular disease and the various physiological processes that can develop in response to or in association with these conditions. There is a further need for an understanding of the role of CTGF in ocular disorders in general, and in the physiological pathways common to many of these disorders. The present disclosure answers these needs by identifying the role of CTGF in various processes associated with the development and progression of ocular disease, and by providing agents for use in methods for inhibiting and preventing these processes.. The disclosure further addresses existing needs by providing agents for use in the treatment, pretreatment, and prevention of ocular diseases and associated conditions.

### SUMMARY OF THE INVENTION

The present disclosure relates to methods for inhibiting or preventing ocular processes. The invention provides the agents of claim 1 and the uses of claim 14.

In various embodiments, the subject is a cell or an animal, preferably a mammal, and most preferably a human.

In one aspect, the agent is selected from the group consisting of an antibody specifically reactive with CTGF. or a fragment or subunit thereof and that neutralizes the biological activity of CTGF. or a fragment thereof, and an antisense oligonucleotide that inhibits expression and activity of CTGF or fragments thereof. In another aspect, the agent is delivered to the eye. Delivery of the agent to the ocular surface is specifically contemplated. In a preferred aspect, the agent is delivered in an eye drop formulation.

The ocular disorder is selected from proliferative vitreoretinopathy and macular degeneration. In a preferred embodiment, the ocular process is further associated with ocular fibrosis.

In certain embodiments, the agents or medicaments are to be administered prior to, at the time of, or in close association with surgery, in order to treat or to prevent the development of ocular disorders that can develop in association with a surgical event. In various aspects, the surgery is selected from the group consisting of refraction correction surgery, radial keratotomy, LASIK, retinal detachment surgery, corneal transplantation, glaucoma filtration surgery, cataract extraction surgery, lens replacement surgery, vitrectomy, subretinal surgery, and retinal translocation surgery.

The agent or medicament of the invention may be for use in treating or preventing macular degeneration. The macular degeneration can be any form of macular degeneration, including age-related macular degeneration, dry form of macular degeneration, also known as non-exudative, involutional, atrophic macular degeneration, and geographic atrophy when severe, and wet form of macular degeneration, also know as exudative, neovascular, or disciform macular degeneration. In a preferred embodiment, the agent or medicament may be for use in treating or preventing choroidal neovascularization associated with macular degeneration. In a preferred embodiment, the agent or medicament is for use in treating or preventing proliferative vitreoretinopathy.

### BRIEF DESCRIPTION OF THE DRAWINGS

Figures 1A and 1B show expression of connective tissue growth factor mRNA in retinal pigment epithelial cells and in choroidal endothelial cells, respectively.

Figures 2A and 2B show connective tissue growth factor expression in retinal pigment epithelial cells after transforming growth factor-β or vascular endothelial cell growth factor treatment.

Figures 3A and 3B show connective tissue growth factor expression in choroidal endothelial cells after vascular endothelial cell growth factor or transforming growth factor-β treatment.

Figures 4A and 4B show the effects of connective tissue growth factor on attachment of retinal pigment epithelial cells and choroidal endothelial cells to fibronectin.

Figures 5A and 5B show the effects of connective tissue growth factor on retinal pigment epithelial cell and choroidal endothelial cell chemotactic migration.

Figures 6A and 6B show dose-dependent stimulation of retinal pigment epithelial cell migration by connective tissue growth factor and N-terminal fragments of connective tissue growth factor.

Figure 7 shows connective tissue growth factor stimulation of retinal pigment epithelial cell proliferation.

Figures 8A and 8B show connective tissue growth factor stimulation of choroidal endothelial cell tube formation.

Figure 9 shows stimulation of extra domain A containing fibronectin expression by connective tissue growth factor in retinal pigment epithelial cells treated with transforming growth factor-β.

Figure 10 shows fibronectin stimulates expression of connective tissue growth factor in retinal pigment epithelial cells.

Figure 11 shows inhibition of fibronectin-induced connective tissue growth factor expression in retinal pigment epithelial cells by anti-α₅β₁ integrin antibody.

Figure 12 shows connective tissue growth factor expression in human proliferative vitreoretinopathy membranes.

Figures 13A, 13B, 13C, and 13D show connective tissue growth factor expression in human choroidal neovascularization membranes from patients with age-related macular degeneration.

Figure 14 shows connective tissue growth factor and fragments of connective tissue growth factor in vitreous fluid excised from individuals with proliferative vitreoretinopathy.

Figure 15 shows connective tissue growth factor and fragments of connective tissue growth factor in vitreous fluid surgically excised from individuals with choroidal neovascularization and retinal neovascularization.

Figures 16A and 16B show connective tissue growth factor induction of fibrosis in an experimental model of proliferative vitreoretinopathy.

Figure 17 shows connective tissue growth factor expression in vitreous fluid of rabbits with various experimental models of proliferative vitreoretinopathy.

### DETAILED DESCRIPTION OF THE INVENTION

Before the present proteins, nucleotide sequences, and methods are described, it is understood that this disclosure is not limited to the particular methodology, protocols, cell lines, vectors, and reagents described, as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. Thus, for example, reference to "a host cell" includes a plurality of such host cells, reference to the "antibody" is a reference to one or more antibodies and equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are cited for the purpose of describing and disclosing the cell lines, vectors, and methodologies which are reported in the publications which might be used in connection with the invention. Nothing herein is to be construed as an admission that the invention is not entitled to antedate such disclosure by virtue of prior invention.

The practice of the present invention will employ, unless otherwise indicated, conventional methods of chemistry, biochemistry, molecular biology, immunology and pharmacology, within the skill of the art. Such techniques are explained fully in the literature. See, e.g., Gennaro, A.R., ed. (1990) Remington's Pharmaceutical Sciences, 18th ed., Mack Publishing Co.; Colowick et al. Methods In Enzymology, Academic Press, Inc.; Handbook of Experimental Immunology, Vols. I-IV (D.M. Weir and C.C. Blackwell, eds., (1986) Blackwell Scientific Publications); Maniatis et al.(1989) Molecular Cloning: A Laboratory Manual, 2nd edition, Vols. I-III, Cold Spring Harbor Laboratory Press; Ausubel et al.(1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., (1997), Springer Verlag).

### Definitions

The terms "ocular disorder" or "ocular disease" are used inclusively and refer to any condition deviating from normal. In particular, the terms "ocular disorder" or "ocular disease" refer to any abnormality of the eye. Ocular disorders can result from any acute or chronic condition or event affecting the eye, including injury, trauma, disease, surgery, etc. Ocular disorders may be associated with various structures in the eye, such as, for example, the retina, macula, vitreous humor, aqueous humor, lens, cornea, sclera, choroid, ciliary body, iris, optic disc, papilla, fovea, or any other portion of the eye. Additionally, ocular disorders can be associated with various ocular cells, such as RPE cells, retinal and choroidal endothelial cells, trabecular meshwork cells, fibroblasts, corneal fibroblasts, glial cells, Müller cells, epithelial cells, such as, for example, lens epithelial cells, corneal epithelial cells, etc.

Ocular disorders or diseases can be associated with any one or more of various ocular processes, including, for example, neovascularization, cell proliferation, cell migration, inflammation, extracellular matrix production and deposition (*e.g*., fibrosis), etc. Examples of ocular disorders include, *e.g*., proliferative vitreoretinopathy, non-proliferative diabetic retinopathy, proliferative diabetic retinopathy, diabetic macular edema, dry macular degeneration, wet macular degeneration, age-related macular degeneration, pigment epithelial detachment associated with macular degeneration, Stargardt's Disease (juvenile macular degeneration), glaucoma, including primary open angle and secondary glaucoma, neovascular glaucoma with proliferative diabetic retinopathy, wound healing associated with glaucoma filtering surgery, idiopathic preretinal fibrosis, subretinal fibrosis, uveal disorders, such as, for example, uveitis syndrome and inflammation of the uvea, idiopathic sclerosing inflammation of the orbit, multifocal fibrosclerosis with orbital involvement, cicatricial pemphigoid with ocular manifestations, systemic lupus with central serous chorioretinopathy, Coats disease with hyperplastic primary vitreous, Vogt-Koyanagi-Harada disease, macular edema, retinal detachment and associated complications, including complications due to retinal detachment surgery, cataracts, macular holes, retinal tears, acute retinal necrosis syndrome, traumatic chorioretinopathies, Purtscher's retinopathy (contusion), retinal edema, corneal epithelial wounds, corneal scarring, corneal haze complicating laser-assisted *in situ* keratomileusis (LASIK), Sjogren's syndrome, corneal neovascularization, iris neovascularization, choroid neovascularization, vitreous neovascularization, ocular neovascularization, ocular inflammation, retinal degenerations, retinal ischemia, vitreoretinal disorders, ocular fibrosis of unknown etiology, sickle cell retinopathy, retinopathy of prematurity, retinal detachment, ocular ischemia, ocular trauma, and radiation retinopathy.

"Fibrosis" is used herein in its broadest sense referring to any excess production or deposition of extracellular matrix proteins. Fibrosis includes any abnormal processing of fibrous tissue, or fibroid or fibrous degeneration. Fibrosis can result from various injuries or diseases, and typically involves the abnormal production, accumulation, or deposition of extracellular matrix components, including overproduction and increased deposition of, for example, collagen and fibronectin. The term "ocular fibrosis" refers to fibrosis affecting the eye or some portion thereof.

"Ocular cells" are cells associated with any structure of the eye, internal or external, including endothelial cells, epithelial cells, fibroblasts, glial cells, retinal pigment epithelial cells, retinal endothelial cells, choroidal endothelial cells, lens epithelial cells, corneal epithelial cells, trabecular meshwork cells, pericytes, astrocytes, microglial cells, perivascular glial cells, Müller cells, perivascular astrocytes, rods, cones, ganglion cells, bipolar cells, etc.

"Connective tissue growth factor" or "CTGF" refers to CTGF derived from any species, preferably a mammalian species, including rat, rabbit, bovine, ovine, porcine, murine, and equine species, and most preferably the human species, and from any source, whether natural, synthetic, semi-synthetic, or recombinant. Connective tissue growth factor has been reported and described previously. (See, *e.g*., U.S. Patent No. 5,408,040; Bradham et al. 1991, J. Cell Biology 114:1285-1294.) In one aspect, "connective tissue growth factor" or "CTGF" refers to a polypeptide sequence comprising at least a portion of the N-terminal fragment of CTGF. In another aspect, "connective tissue growth factor" or "CTGF" refers to a polypeptide sequence comprising at least a portion of the C-terminal fragment of CTGF. The term "N-terminal fragment" used in reference to CTGF means any polypeptide comprising sequence derived from the amino-terminal portion of a CTGF polypeptide, or to any variants, or fragments thereof. The term "C-terminal fragment" used in reference to CTGF means any polypeptide comprising sequence derived from the carboxy-terminal portion of a CTGF polypeptide, or to any variants, of fragments thereof. (See, *e.g*., International Publication No. WO 00/35939; International Publication No. WO 00/35936; and U.S. Application Serial No. 10/245,977.)

The phrase "CTGF-associated ocular disorders" as used herein refers to ocular conditions and diseases associated with abnormal or inappropriate expression or activity of CTGF or fragments thereof. The term specifically contemplates diseases and disorders associated with ocular cell proliferation, ocular cell migration, extracellular matrix production or deposition in the eye, including ocular fibrosis and scarring, diseases and disorders associated with inflammation in the eye, and ocular angiogenesis and neovascularization.

The proliferative processes and disorders referred to herein include pathological states characterized by the continual multiplication of cells resulting in an overgrowth of a cell population within a tissue. The cell populations are not necessarily transformed, tumorigenic, or malignant cells, but can include normal cells as well. For example, CTGF may be involved pathologically by inducing a proliferative response in various cells of the retina, such as retinal pigment epithelial cells, or by stimulating neovascularization.

The terms "angiogenesis" and "neovascularization" refer to the formation or growth of new or existing capillaries or blood vessels.

The term "agonist" as used in reference to CTGF refers to a molecule which increases or prolongs the extent or duration of the effect of the biological or immunological activity or expression of a particular molecule, *e.g*., CTGF or fragments of CTGF. Agonists may include proteins, nucleic acids, carbohydrates, antibodies, small molecules, or any other molecules which increase the effect of CTGF or fragments thereof.

The term "antagonist" as used in reference to CTGF refers to a molecule which decreases the extent or duration of the effect of the biological or immunological activity or expression of a particular molecule, *e.g*., CTGF or fragments of CTGF. Antagonists may include proteins, nucleic acids, carbohydrates, antibodies, small molecules, or any other molecules which decrease the effect of CTGF or fragments thereof.

The term "antibody" refers to immunoglobulins or antibodies, monoclonal or polyclonal, obtained from any source. Such sources can include a cell line, or an animal such as mouse, rat, rabbit, chicken, turkey, goat, horse, human, etc. Antibodies may also be obtained from genetically modified cells, or transgenic plants or animals engineered to make antibodies that are not endogenous to the host. An antibody may be of any isotype including, *e.g*., IgA, IgD, IgE, IgG-1, IgG-2, IgG-3, IgG-4, or IgM, etc. As used herein, "antibody" includes intact molecules and fragments thereof, such as Fab, F(ab')₂, and Fv fragments, capable of binding the epitopic determinant, chimeric antibodies, *e.g*., bivalent and trivalent antibodies, that bind to one or more unique antigen(s).

The term "antisense" refers to any composition containing a nucleic acid sequence which is complementary to the "sense" strand of a specific nucleic acid sequence. Antisense molecules may be produced by any method available in the art including by synthesis or transcription. Once introduced into a cell, the complementary nucleotides can combine with natural sequences produced by the cell to form duplexes and to block either transcription or translation of the corresponding natural sequences.

"Amino acid sequence" or "polypeptide" or "polypeptides," as these terms are used herein, refer to oligopeptide, peptide, polypeptide, or protein sequences, and fragments thereof, and to naturally occurring or synthetic molecules. Polypeptide or amino acid fragments are any portion of a polypeptide which retains at least one structural and/or functional characteristic of the polypeptide. CTGF fragments include any portion of a CTGF polypeptide sequence which retains at least one structural or functional characteristic of CTGF. Where "amino acid sequence" refers to the polypeptide sequence of a naturally occurring protein molecule, "amino acid sequence" and like terms are not meant to limit the amino acid sequence to the complete native sequence associated with the protein molecule in question.

The terms "nucleic acid" or "polynucleotide" or "polynucleotides" refer to oligonucleotides, nucleotide sequences, or polynucleotides, or any fragments thereof, and to DNA or RNA of natural or synthetic origin which may be single- or double-stranded and may represent the sense or antisense strand, to peptide nucleic acid (PNA), or to any DNA-like or RNA-like material, natural or synthetic in origin. Polynucleotide fragments are any portion of a polynucleotide sequence that retains at least one structural or functional characteristic of the polynucleotide. Polynucleotide fragments can be of variable length, for example, greater than 60 nucleotides in length, at least 100 nucleotides in length, at least 1000 nucleotides in length, or at least 10,000 nucleotides in length.

"Altered" polynucleotides include those with deletions, insertions, or substitutions of different nucleotides resulting in a polynucleotide that encodes the same or a functionally equivalent polypeptide. Included within this definition are sequences displaying polymorphisms that may or may not be readily detectable using particular oligonucleotide probes or through deletion of improper or unexpected hybridization to alleles, with a locus other than the normal chromosomal locus for the subject polynucleotide sequence.

"Altered" polypeptides may contain deletions, insertions, or substitutions of amino acid residues which produce a silent change and result in a functionally equivalent polypeptide. Deliberate amino acid substitutions may be made on the basis of similarity in polarity, charge, solubility, hydrophobicity, hydrophilicity, and/or the amphipathic nature of the residues as long as the biological or immunological activity of the encoded polypeptide is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine.

A polypeptide or amino acid "variant" is an amino acid sequence that is altered by one or more amino acids from a particular amino acid sequence. A polypeptide variant may have conservative changes, wherein a substituted amino acid has similar structural or chemical properties to the amino acid replaced, *e.g*., replacement of leucine with isoleucine. A variant may also have non-conservative changes, in which the substituted amino acid has physical properties different from those of the replaced amino acid, *e.g*., replacement of a glycine with a tryptophan. Analogous minor variations may also include amino acid deletions or insertions, or both. Preferably, amino acid variants retain certain structural or functional characteristics of a particular polypeptide. Guidance in determining which amino acid residues may be substituted, inserted, or deleted may be found, for example, using computer programs well known in the art, such as LASERGENE software (DNASTAR Inc., Madison, WI).

A polynucleotide variant is a variant of a particular polynucleotide sequence that preferably has at least about 80%, more preferably at least about 90%, and most preferably at least about 95% polynucleotide sequence similarity to the particular polynucleotide sequence. It will be appreciated by those skilled in the art that as a result of the degeneracy of the genetic code, a multitude of variant polynucleotide sequences encoding a particular protein, some bearing minimal homology to the polynucleotide sequences of any known and naturally occurring gene, may be produced. Thus, the invention contemplates each and every possible variation of polynucleotide sequence that could be made by selecting combinations based on possible codon choices. These combinations are made in accordance with the standard codon triplet genetic code, and all such variations are to be considered as being specifically disclosed.

A "deletion" is a change in an amino acid or nucleotide sequence that results in the absence of one or more amino acid residues or nucleotides.

The terms "insertion" or "addition" refer to a change in a polypeptide or polynucleotide sequence resulting in the addition of one or more amino acid residues or nucleotides, respectively, as compared to the naturally occurring molecule.

The term "functional equivalent" as it is used herein refers to a polypeptide or polynucleotide that possesses at least one functional or structural characteristic of a particular polypeptide or polynucleotide. A functional equivalent may contain modifications that enable the performance of a specific function. The term "functional equivalent" is intended to include fragments, mutants, hybrids, variants, analogs, or chemical derivatives of a molecule.

The term "sample" is used herein in its broadest sense. Samples may be derived from any source, for example, from bodily fluids, secretions, tissues, cells, or cells in culture, including, but not limited to, vitreous humour, aqueous humor, tears, saliva, blood, urine, serum, plasma, synovial fluid, cerebral spinal fluid, amniotic fluid, and organ tissue (*e.g*., biopsied tissue); from chromosomes, organelles, or other membranes isolated from a cell; from genomic DNA, cDNA, RNA, mRNA, etc.; and from cleared cells or tissues, or blots or imprints from such cells or tissues. Samples may be derived from any source, such as, for example, a cell, a human subject, a non-human mammalian subject, etc. Also contemplated are samples derived from any animal model of disease. A sample can be in solution or can be, for example, fixed or bound to a substrate. A sample can refer to any material suitable for testing for the presence of CTGF or fragments of CTGF or suitable for screening for molecules that bind to CTGF or fragments thereof. Methods for obtaining such samples are within the level of skill in the art.

As used herein, the terms "extracellular matrix," and "extracellular matrix proteins" refer broadly to non-cellular matrix, typically composed of proteins, glycoproteins, complex carbohydrates, and other macromolecules. Extracellular matrix proteins include, for example, collagens, such as collagen types I and IV, fibronectin, laminin, and thrombospondin.

Tho term "microarray" refers to any arrangement of molecules, *e.g*. nucleic acids, amino acids, antibodies, etc., on a substrate. The substrate can be any suitable support, *e.g*., beads, glass, paper, nitrocellulose, nylon, or any appropriate membrane, etc. A substrate can be any rigid or semi-rigid support including, but not limited to, membranes, filters wafers, chips, slides, fibers, beads, including magnetic or nonmagnetic beads, gels, tubing, plates, polymers, microparticles, capillaries, etc. The substrate can provide a surface for coating and/or can have a variety of surface forms, such as wells, pins, trenches, channels, and pores, to which the nucleic acids, amino acids, etc., may be bound.

The terms "proliferative vitreoretinopathy" or "PVR" refer a variety of intraocular pathologies, including epiretinal membranes, subretinal stands, etc., and retinal detachment in combination with star folds, vitreous traction, anterior loop traction, etc. PVR also includes any intraocular cellular proliferation associated with any degree of retinal detachment

### The Invention

The present disclosure relates to methods for inhibiting or preventing ocular processes associated with, *e.g.,* cell proliferation, cell migration, neovascularization, inflammation, fibrosis, etc. The inventor provides the agents of claim 1 and the uses of claim 14.

Acute or chronic conditions and events, such as, for example, injury, trauma, disease, surgery, etc., are associated with development and progression of one or more ocular processes that can lead to various structural, pathological, physiological, and cellular changes within the eye. Such processes include, *e.g*., alterations in basement membrane matrix composition; increased production and deposition of extracellular matrix leading to fibrosis and scar formation; neovascularization; increased capillary permeability; ocular cell hyperplasia; ocular cell proliferation; ocular cell migration; development of ocular membranes, such as cellular, fibrotic, subretinal, epiretinal, vitreous, choroidal neovascular membranes, etc.

These ocular processes are associated with many ocular disorders and diseases and can often occur in association with each other. The interrelatedness of multiple ocular processes is evident, for example, in PVR, in which RPE cell proliferation and RPE cell migration are associated with the formation of cellular and fibrotic membranes within the vitreous (vitreous membranes) or on the inner or outer surfaces of the retina (epiretinal membranes). Diabetic retinopathy is associated with damage or alterations in the inner blood vessels of the retina, resulting in hemorrhage and swelling, leaking of fluid into the retina, and new growth of blood vessels on the surface of the retina and into the vitreous. CNV is associated with increased choroidal epithelial cell adhesion, migration, and proliferation; extracellular matrix overproduction; and development of a fibrovascular subretinal membrane. It is understood, therefore, that various ocular processes are interrelated, and thus provide therapeutic pathways common to a wide range of disorders and diseases affecting the eye. Effective methods of inhibiting and preventing these processes, and treating and preventing the associated disorders, thus have broad application.

The present disclosure relates to the discovery that CTGF is a central factor in the initiation, development, and progression of various ocular processes. Further, the present invention demonstrates that expression and activity of CTGF are associated with the onset and extent of ocular disorders in general, and, in particular, with disorders associated with the processes enumerated above.

### CTGF in Ocular Processes and in Ocular Disorders

### Involvement of CTGF in ocular processes and ocular disorders

The present disclosure demonstrates increased expression of CTGF in various ocular processes and associated diseases and conditions. For example, CTGF expression was elevated in vitreous of patients diagnosed with certain ocular diseases, and in animal models of ocular disorders. (See, *e.g*., Examples 14, 15, and 22.) The level of CTGF was highest in vitreous samples taken from patients with ocular processes commonly associated with wound healing, such as, for example, neovascularization and fibrosis. (See, *e.g*., Examples 14 and 15.) Increased CTGF expression was observed in vitreous samples obtained from patients with retinal detachment without PVR, retinal detachment with PVR, epiretinal membranes, macular hole, CNV, retinal neovascularization, proliferative diabetic retinopathy, and age-related macular degeneration. (See, *e.g*., Example 14.)

Additionally, the inventors found CTGF expression is elevated in ocular tissues obtained from individuals having ocular disorders, such as PVR, diabetic retinopathy, and CNV, including CNV associated with macular degeneration. For example, immunohistochemical data show CTGF was expressed in PVR membranes surgically excised from patients with retinal detachment. (See, *e.g*., Example 12.) Increased expression of CTGF was observed in CNV membranes obtained from subjects with clinically diagnosed age-related macular degeneration. (See, *e.g*., Example 13.) Within isolated CNV membranes, CTGF was predominantly localized to neovascular membranes as well as associated with cells surrounding blood vessels. CTGF is thus associated with neovascularization, ocular fibrosis, and ocular membrane formation.

The present inventors further demonstrated expression of CTGF in various ocular cells, including, *e.g*., RPE cells, CECs, and retinal Müller cells. The present invention shows that CTGF expression in various ocular cells is differentially regulated by various growth factors and extracellular matrix components associated with various ocular disorders and ocular processes. Specifically, TGFβ stimulated CTGF expression in RPE cells, and VEGF stimulated CTGF expression in CECs. (See, *e.g*., Example 2.) Fibronectin, an extracellular matrix component associated with ocular cell migration and proliferation; stimulated CTGF expression in RPE cells. (See, *e.g*., Example 10.)

### CTGF induces ocular fibrosis

The present disclosure provides the first demonstration that CTGF induces ocular fibrosis (increased extracellular matrix production and deposition). The present inventors found that CTGF induced pathologic ocular fibrosis *in vivo* in validated animal models of ocular disease and that addition of CTGF to vitreous stimulated development and progression of ocular fibrosis. (See, *e.g*., Examples 17 and 19.) Ocular fibrosis induced by CTGF was associated with transformation of epiretinal RPE membranes into paucicellular, myofibroblastic, and densely fibrotic epiretinal membranes. CTGF injected into the vitreous of a rabbit with a mild degree of PVR resulted in the formation of densely fibrotic membranes and development and progression of ocular fibrosis. (See, *e.g*., Examples 17 and 19.) Further, the present inventors showed that CTGF stimulated extracellular matrix production by demonstrating that CTGF induced expression of fibronectin, a component of extracellular matrix, in RPE cells. (See, *e.g*., Example 9.) These results provide direct evidence that CTGF induces ocular fibrosis, and thus CTGF represents a target for inhibition of the development and progression of the fibrotic process in the eye (*i.e*., ocular fibrosis) and associated diseases and disorders.

The present disclosure demonstrates that CTGF stimulated extracellular matrix production, a feature of ocular fibrosis, in ocular cells. Specifically, the present inventors showed that CTGF induced the expression of fibronectin in RPE cells.
(See, *e.g*., Example 9.)

### CTGF induces ocular angiogenesis and ocular neovascularization

The present inventors also provide evidence that CTGF induces ocular angiogenesis and ocular neovascularization. Neovascularization is associated with various ocular disorders and ocular processes, including, for example, the development and progression of ocular membranes, proliferative neovascularization, and choroidal neovascularization.

Using an *in vitro* tube formation assay, in which endothelial cells undergo proliferation, migration, and differentiation, the present inventors demonstrated that monolayer cultures of CECs exhibited increased tube formation in response to CTGF, thus establishing the involvement of CTGF in ocular angiogenesis and neovascularization.
(See, *e.g*., Example 8.)

Further findings showed that CTGF was predominantly localized to neovascular membranes as well as associated with cells surrounding blood vessels in CNV membranes. (See, *e.g*., Example 13.) The present inventors thus were able to establish the involvement of CTGF in ocular angiogenesis and neovascularization, and neovascular membrane formation.

### CTGF stimulates ocular cell proliferation and migration

In many ocular disorders, proliferation and migration of ocular cells are associated with development and progression of various ocular processes and associated diseases and disorders. The present invention demonstrates that CTGF stimulates proliferation and migration of ocular cells critically involved in the development and progression of ocular processes, such as, for example, cell proliferation, cell migration, neovascularization, and fibrosis. For example, RPE cell proliferation was stimulated by CTGF. (See, *e.g*., Examples 7.) Additionally, CTGF stimulated migration of RPE cells, CECs, and retinal Müller cells. (See, *e.g.,* Examples 6 and 11.)

### Methods for inhibiting or preventing ocular processes and ocular disorders

The present disclosure relates to methods for inhibiting or preventing ocular processes. The invention provides the agents of claim 1 and the uses of claim 14.

Disclosed herein is an agent that decreases expression or activity of CTGF or fragments thereof for use in inhibiting or preventing an ocular process associated with CTGF in a subject The agent can be applied *in vivo* or *in vitro.* In various embodiments, the subject is a cell or an animal, preferably a mammal, and most preferably a human.

In one aspect, the agent is selected from the group consisting of an antibody specifically reactive with CTGF or a fragment or subunit thereof and that neutralizes the biological activity of CTGF or a fragment thereof, an antisense oligonucleotide that inhibits expression and activity of CTGF or fragments thereof. In another aspect, the agent is delivered to the eye. Delivery of the agent to the ocular surface is specifically contemplated. In a preferred aspect, the agent is delivered in an eye drop formulation. In another aspect, the agent is delivered as an ointment.

In preferred embodiments, the ocular process is further associated with an ocular cell. In certain embodiments, the ocular cell is selected from the group consisting of an endothelial cell, an epithelial cell, a fibroblast, a glial cell, a retinal pigment epithelial cell, a retinal endothelial cell, a choroidal endothelial cell, a lens epithelial cell, a corneal epithelial cell, a trabecular meshwork cell, a pericyte, an astrocyte, a microglial cell, a perivascular glial cell, a Müller cell, a perivascular astrocyte, a rod, a cone, a ganglion cell, and a bipolar cell.

In another embodiment, the ocular process is further associated with an ocular structure. In various embodiments the ocular structure is selected from the group consisting of the retina, retinal pigment epithelium layer, choroid, macular, cornea, lens, iris, sclera, trabecular meshwork, aqueous, aqueous chamber, vitreous, ciliary body, optic disc, papilla, and fovea.

Agents for use in inhibiting or preventing an ocular process associated with CTGF, wherein the agent decreases expression or activity of CTGF or fragments thereof and wherein the ocular process is further associated with an ocular disorder, are specifically contemplated. Application of the present agents to any CTGF-associated ocular disorder is specifically contemplated. In particular embodiments, the ocular disorder is selected from the group consisting of glaucoma, cataract, choroidal neovascularization, retinal detachment, proliferative vitreoretinopathy, macular degeneration, diabetic retinopathy, corneal scaning, and corneal haze. In a preferred embodiment, the ocular process is further associated with ocular fibrosis.

Agents for use inhibiting or preventing ocular extracellular matrix production or deposition, in a subject wherein said agents decrease expression or activity of CTGF or fragments thereof, are specifically disclosed herein. Agents for use in inhibiting or preventing ocular scarring, in a subject wherein said agents decrease, expression or activity of CTGF or fragments thereof, are specifically discloesd. In one embodiment, the agents are for use in inhibiting or preventing ocular neovascularization, in a subject wherein said agents decrease expression or activity of CTGF or fragments thereof. In further embodiments, the neovascularization is retinal neovascularization, choroidal neovascularization, neovascularization of the iris, or trabecular neovascularization. Agents for use in inhibiting or preventing ocular inflammation, in a subject wherein said agents decrease expression or activity of CTGF or fragments thereof, are also disclosed.

Also disclosed is an agent for use in a inhibiting or preventing ocular cell proliferation in a subject agent wherein the decreases expression or activity of CTGF or fragments thereof. In various embodiments, the ocular cell proliferation is selected from the group consisting of epithelial cell proliferation, endothelial cell proliferation, retinal pigment epithelial cell proliferation, and choroidal endothelial cell proliferation.

Also disclosed is an agent for use in inhibiting or preventing ocular cell migration in a subject wherein the agent decreases expression or activity of CTGF or fragments thereof. In particular aspects, the ocular cell migration is selected from the group consisting of epithelial cell migration, retinal pigment epithelial cell migration, endothelial cell migration, and choroidal endothelial cell migration.

Also disclosed is an agent for use in inhibiting or preventing an ocular process associated with CTGF in a subject, wherein the agent decreases expression or activity of CTGF or fragments thereof and wherein the ocular process is further associated with surgery. In various aspects, the surgery is selected from the group consisting of refraction correction surgery, radial keratotomy, LASIK, retinal detachment surgery, corneal transplantation, glaucoma filtration surgery, cataract extraction surgery, lens replacement surgery, vitrectomy, subretinal surgery, and retinal translocation surgery.

### Agents for use in treating and preventing ocular processes and ocular disorders

Agents for use in treating or preventing ocular disorders are also disclosed. In one embodiment, the agent for use in treating or preventing an ocular disorder in a subject wherein the agent decreases expression or activity of CTGF or fragments thereof In another embodiment the agent is for use in treating or preventing ocular fibrosis in a subject wherein the agent decreases expression or activity of CTGF or fragments thereof.

Disclosed herein is an agent for use in treating or preventing an ocular disorder associated with neovascularization in a subject wherein the agent decreases expression or activity of CTGF or fragments thereof. In various aspects, the neovascularization is retinal neovascularization, choroidal neovascularization, neovascularization of the iris, or trabecular neovascularization. In a particular aspect, a method for treating or preventing choroidal neovascularization associated with macular degeneration is specifically contemplated.

Agents for use in treating or preventing an ocular disorder associated with extracellular matrix production or deposition in a subject wherein said agents decrease expression or activity of CTGF or fragments thereof, is further disclosed, as are agents for use in treating or preventing an ocular disorder associated with inflammation in a subject wherein said agents decrease expression or activity of CTGF or fragments thereof.

Also disclosed are agents for use in terating or preventing an ocular disorder associated with cell proliferation in a subject wherein said agents decrease expression or activity of CTGF or fragments thereof. In various embodiments, the cell is selected from the group consisting of an endothelial cell, an epithelial cell, a fibroblast, a glial cell, a retinal pigment epithelial cell, a retinal endothelial cell, a choroidal endothelial cell, a lens epithelial cell, a corneal epithelial cell, a trabecular meshwork cell, a pericyte, an astrocyte, a microglial cell, a perivascular glial cell, a Müller cell, a perivascular astrocyte, a rod, a cone, a ganglion cell, and a bipolar cell.

Also disclosed are agents for use in treating or preventing an ocular disorder associated with cell migration in a subject wherein said agents decrease expression or activity of CTGF or fragments thereof. In certain embodiments, the cell is selected from the group consisting of an endothelial cell, an epithelial cell, a fibroblast, a glial cell, a retinal pigment epithelial cell, a retinal endothelial cell, a choroidal endothelial cell, a lens epithelial cell, a corneal epithelial cell, a trabecular meshwork cell, a pericyte, an astrocyte, a microglial cell, a perivascular glial cell, a Müller cell, a perivascular astrocyte, a rod, a cone, a ganglion cell, and a bipolar cell. In certain aspects, the agents are for use in treating ocular disorders associated with altered ocular cell migration, proliferation, chemotaxis, or attachment.

Agents for use in treating or preventing a disorder associated with formation or alteration of an ocular membrane in a subject wherein the agents decrease expression or activity of CTGF or fragments thereof, are specifically contemplated. In various aspects, the ocular membrane is selected from the group consisting of an epiretinal membrane, a subretinal membrane, a vitreous membrane, a cellular membrane, a choroidal neovascularization membrane, and a fibrotic membrane. In a particular, embodiment, the agents are or use method in maintaining or improving vision in a subject wherein the agents decrease expression or activity of CTGF or fragments thereof.

In certain embodiments, the agents for use in treating or preventing an ocular disorder are to be administered prior to, at the time of or in close association with surgery, in order to treat or to prevent the development of ocular disorders that can develop in association with a surgical event. In various aspects, the surgery is selected from the group consisting of refraction correction surgery, radial keratotomy, LASIK, retinal detachment surgery, corneal transplantation, glaucoma filtration surgery, cataract extraction surgery, lens replacement surgery, vitrectomy, subretinal surgery, and retinal translocation surgery.

The present invention provides an agent for use in a method for treating or preventing macular degeneration. Any of the agents for use in the methods described in the present invention can be applied to treat or prevent any form of macular degeneration, including age-related macular degeneration, dry form of macular degeneration, also known as non-exudative, involutional, atrophic macular degeneration, and geographic atrophy when severe, and wet form of macular degeneration, also know as exudative, neovascular, or disciform macular degeneration. In a preferred embodiment, the agent is for use in treating or preventing choroidal neovascularization associated with macular degeneration. Disclosed herein is a method for treating or preventing diabetic retinopathy, including proliferative diabetic retinopathy and non-proliferative diabetic retinopathy. In a preferred embodiment, the present invention provides an agent for use in a method for treating or preventing proliferative vitreoretinopathy. Disclosed herein are agents for use in treating or preventing retinal detachment, glaucoma, cataracts, corneal scarring; and corneal haze.

Also disclosed in an agent for use maintaining or improving vision in a subject wherein the agent decreases expression or activity of CTGF or fragments thereof.

### Pharmaceutical formulations and routes of administration

The present invention relates to the use of compounds or agents that modulate the activity or expression of CTGF are administered, for example, *in vivo*, to affect the activity of CTGF. These agents can be delivered directly or in pharmaceutical compositions along with suitable carriers or excipients, as well known in the art. An effective amount of an agent that inhibits the activity or expression of CTGF or fragments of CTGF may be administered to a subject in need of treatment. In a preferred embodiment, the subject is a mammalian subject, and in a most preferred embodiment, the subject is a human subject. Various formulations and drug delivery systems are available in the art. (See, *e.g.*, Remington's Pharmaceutical Sciences, *supra.)* In one aspect, the agent is selected from the group consisting of an antibody, and an antisense oligonucleotide. In another aspect, the agent is delivered to the eye. In one embodiment the agent is delivered to the ocular surface. In a preferred aspect, the agent is delivered in an eye drop formulation. In another aspect, the agent is delivered as an ointment.

Suitable routes of administration may, for example, include oral, rectal, transmucosal, nasal, or intestinal administration and parenteral delivery, including intramuscular, subcutaneous, intramedullary injections, as well as intrathecal, direct intraventricular, intravenous, intraperitoneal, intranasal, or intraocular injections. The agent maybe administered in a local or a systemic manner. For example, a suitable agent can be delivered via injection or in a targeted drug delivery system, such as depot, catheter, or sustained release formulation. Local ocular administration can include, for example, intraocular, intravitreal, subretinal, subscleral, subconjunttival, subtenon, or intrascleral, injection or application. Suitable agents can also be applied directly to the cornea or sclera.

For any composition or agent for use in the present methods of treatment, a therapeutically effective dose can be estimated initially using a variety of techniques well known in the art. For example, in a cell culture assay, a dose can be formulated in animal models to achieve a circulating concentration range that includes the IC₅₀ as determined in cell culture. Where inhibition of CTGF expression or activity is desired, for example, the concentration of the test agent that achieves a half-maximal inhibition of CTGF activity can be determined. Dosage ranges appropriate for human subjects can be determined, for example, using data obtained from cell culture assays and other animal studies. A therapeutically effective dose of an agent refers to that amount of the agent that results in amelioration of symptoms or a prolongation of survival in a subject.

### Antibodies

Antibodies directed to CTGF or fragments of CTGF may be generated using methods well known in the art. Such antibodies may include, but are not limited to, polyclonal, monoclonal, chimeric, and single chain antibodies, as well as Fab fragments, including F(ab')₂ and Fᵥ fragments. Fragments can be produced, for example, by a Fab expression library. Neutralizing antibodies, *i.e*., those which inhibit CTGF activity, are especially preferred for therapeutic use.

Methods for the production of antibodies are well known in the art. For example, various hosts, including goats, rabbits, rats, mice, chickens, turkeys, humans, and others, may be immunized by injection with the target polypeptide or any immunogenic fragment or peptide thereof. Techniques for *in vivo* and *in vitro* production of either monoclonal or polyclonal antibodies are well known in the art. (See, *e.g*., Pound (1998) Immunochemical Protocols, Humana Press, Totowa NJ; Harlow and Lane (1988) Antibodies: A Laboratory Manual, Cold Spring Harbor Laboratory, New York; Goding (1986) Monoclonal Antibodies: Principles and Practice, 2nd Edition, Academic Press; Schook (1987) Monoclonal Antibody Production Techniques and Applications, Marcel Dekker, Inc.)

Antibodies as described above could also be used to identify CTGF, or fragments or subunits thereof, in a sample, *e.g*., from blood, biopsied tissue, vitreous, tears, etc. The amount of CTGF or fragments of CTGF present could be determined, for example, by quantitative image analysis, ELISA, or imuunohistochemical techniques. CTGF mRNA levels could also be determined, *e.g*., by reverse transcriptase polymerase chain reaction (RT-PCR), using portions of the biopsied tissue, or by other methods well known in the art. In particular, in this method, mRNA from a tissue sample, in total, or that specific for CTGF or fragments or subunits thereof, could be transcribed to DNA and then amplified through PCR using specific primer sequences. Quantitation of mRNA corresponding to CTGF could be determined, for example, by a competition reaction using equal volumes of the patient sample run against a series of decreasing known concentrations, *e.g*., of a mimic or mutant cDNA fragment.

The present disclosure contemplates the use of antibodies specifically reactive with CTGF or fragments or subunits thereof that neutralize the biological activity of CTGF or fragments of CTGF. The antibody administered in the method can be an intact antibody or antigen binding fragments thereof, such as Fab, F(ab')2, and Fᵥ fragments, which are capable of binding the epitopic determinant. The antibodies used in the method can be polyclonal or, more preferably, monoclonal antibodies. Monoclonal antibodies with different epitopic specificities are made from antigen-containing fragments of the protein by methods well known in the art. (See Ausubel *et al., supra.)*

In the present disclosure, therapeutic applications include those using "human" or "humanized" antibodies directed to CTGF or fragments thereof. Humanized antibodies are antibodies, or antibody fragments, that have the same binding specificity as a parent antibody, (*i.e.*, typically of mouse origin) and increased human characteristics. Humanized antibodies may be obtained, for example, by chain shuffling or by using phage display technology. For example, a polypeptide comprising a heavy or light chain variable domain of a non-human antibody specific for CTGF is combined with a repertoire of human complementary (light or heavy) chain variable domains. Hybrid pairings specific for the antigen of interest are selected. Human chains from the selected pairings may then be combined with a repertoire of human complementary variable domains (heavy or light) and humanized antibody polypeptide dimers can be selected for binding specificity for an antigen. Techniques described for generation of humanized antibodies that can be used in the method of the present invention are disclosed in, for example, U.S. Patent Nos. 5,565,332; 5,585,089; 5,694,761; and 5,693,762. Furthermore, techniques described for the production of human antibodies in transgenic mice are described in, for example, U.S. Patent Nos. 5,545,806 and 5,569,825.

### Antisense

The present disclosure provides for a therapeutic approach which uses antisense technology to inhibit expression and activity of CTGF or fragments thereof. Specifically, a therapeutic approach which directly interrupts the transcription of DNA encoding CTGF or translation of CTGF mRNA could be used to alter the expression and activity of CTGF and of fragments of CTGF.

Antisense technology relies on the modulation of expression of a target protein through the specific binding of an antisense sequence to a target sequence encoding the target protein or directing its expression. (See, *e.g*., Agrawal, S., ed. (1996) Antisense Therapeutics, Humana Press Inc., Totawa NJ; Alama et al. (1997) Pharmacol Res 36(3):171-178; Crooke (1997) Adv Pharmacol 40:1-49; Lavrosky et al. (1997) Biochem Mol Med 62(1):11-22.)

Delivery of antisense sequences can be accomplished in a variety of ways, such as, for example, through intracellular delivery using an expression vector or direct administration of antisense oligonucleotides.

Delivery of antisense therapies and the like can be achieved intracellularly through using a recombinant expression vector such as a chimeric virus or a colloidal dispersion system which, upon transcription, produces a sequence complementary to at least a portion of the cellular sequence encoding the target protein. (See, *e.g*., Slater et al. (1998) J Allergy Cli Immunol 102(3):469-475.) Delivery of antisense sequences can also be achieved through various viral vectors, including retrovirus and adeno-associated virus vectors. (See, *e.g*., Miller (1990) Blood 76:271; Uckert and Walther (1994) Pharacol Ther 63(3):323-347.) Vectors which can be utilized for antisense gene therapy as taught herein include, but are not limited to, adenoviruses, herpes viruses, vaccinia, or, preferably, RNA viruses such as retroviruses.

Other gene delivery mechanisms that can be used for delivery of antisense sequences to target cells include colloidal dispersion and liposome-derived systems, artificial viral envelopes, and other systems available to one of skill in the art. (See, *e.g*., Rossi (1995) Br Med Bull 51(1):217-225; Morris et al. (1997) Nucl Acids Res 25(14):2730-2736; Boado et al. (1998) J Pharm Sci 87(11):1308-1315.) For example, delivery systems can make use of macromolecule complexes, nanocapsules, microspheres, beads, and lipid-based systems including oil-in-water emulsions, micelles, mixed micelles, and liposomes.

The following examples explain in the invention in more detail. The following preparations and examples are given to enable those of skill in the art to more clearly understand and to practice the present invention. The present invention, however, is not limited in scope by the exemplified embodiments, which are intended as illustrations of single aspects of the invention.

### EXAMPLES

### Example 1: RPE cells and CECs express CTGF mRNA

Human RPE cells were derived as follows. Human fetal eyes (18 to 26 weeks of gestation) were obtained from the Anatomic Gift Foundation (Woodbine, GA). RPE cells were isolated from human fetal eyes using methods previously described. (Jin et al. (2000) IOVS 41:4324-4332.) RPE cells at passage two to four were used in all studies. RPE cells were cultured in Dulbecco's Eagles Medium (Fisher Scientific, Pittsburgh, PA) supplemented with 10% fetal bovine serum (Gibco/BRL, Gathersbery, MD), 2 mM glutamine, 100 µg/ml streptomycin, and 100 µg/ml penicillin (Sigma Chemical Co., St. Louis, MO). The purity of cultured RPE cells was evaluated by immuno-cytochemical staining using an antibody against cytokeratin (Catalog No. M0821, Dako, Carpinteria, CA), endothelial cell antigen von Willebrand factor (Catalog No. M0616, Dako), and macrophage antigen CD11c (Catalog No. M0732, Dako). Cultured RPE cells used in these studies were typically 99% pure based on immuno-cytochemical staining performed using these cell-type specific markers.

CECs were isolated from bovine eyes using previously described methods. (Hoffmann et al. (1998) Graefes Arch Clin Exp Ophthalmol 236:779-784.) Briefly, magnetic beads (Dynabeads) coated with Lycopersicon esculentum (Sigma Chemical Co.), a specific endothelial cell marker, were used to isolate CECs from bovine eyes. CECs isolated from bovine eyes were confirmed to be vascular endothelial cells by positive immunostaining for von Willebrand factor, as well as by the ability of the isolated CECs to bind dil-acetylated low-density lipoprotein. (Hoffman et al. *supra.)*

Expression of CTGF mRNA was examined in cultured human RPE cells and in cultured bovine CECs using reverse transcriptase polymerase chain reaction (RT-PCR). Poly(A)⁺ RNA was isolated from RPE cells and CECs using a FastTrack Kit (Invitrogen, San Diego, CA), according to methods described by the manufacturer. First-strand cDNA was synthesized at 42°C using M-MLV reverse transcriptase (Gibco/BRL) with an oligo-d(T) primer (Catalog No. C110A, Promega, Madison, WI). PCR was performed using oligonucleotide sequence primers specific for human CTGF or bovine CTGF. The primer sequences used for PCR for amplification of human CTGF sequence from RPE cells were 5'-gcatccgtactcccaaaatc-3' (SEQ ID NO:1) and 5'-cttcttcatgacctcgccgt-3' (SEQ ID NO:2). The primer sequences used for PCR amplification of bovine CTGF sequence from CECs were 5'-gatcataggactgtattagtgc-3' (SEQ ID NO:3) and 5'-ctgacttagagacgaacttg-3' (SEQ ID NO:4). PCR was performed using a Perkin-Elmer/Centur DNA thermal cycler, using the following conditions: denaturation at 94°C for 1 minute; annealing at 60°C for 1 minute; and extension at 72°C for 45 seconds. Following 30 cycles of PCR, the amplified products were resolved on 1.2% agarose gels and viewed following staining of the gel with ethidium bromide. As expected, the use of these primers resulted in CTGF-specific DNA amplification products of 210 base pairs (human CTGF) and 220 base pairs (bovine CTGF) in size.

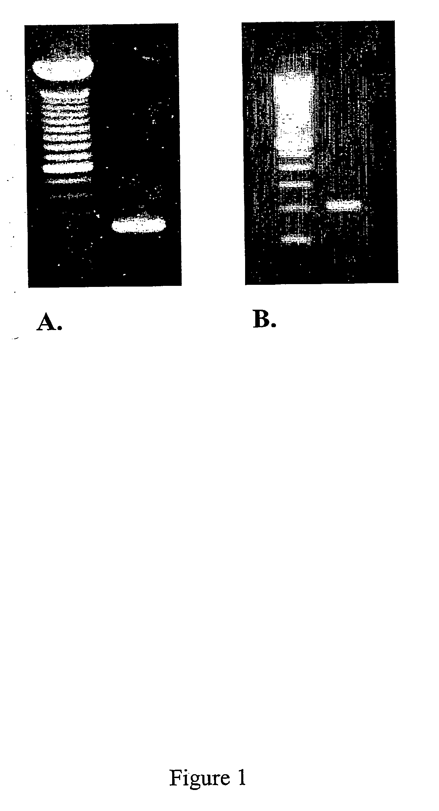

CTGF mRNA was detected in cultured RPE cells and in CECs. As shown in Figure 1A and Figure 1B (right lanes), both RPE cells and CECs were positive for CTGF mRNA expression, as evidenced by the presence of the expected 210 base pair and 220 base pair CTGF RT-PCR amplification products. The molecular weight markers shown in Figure 1A and Figure 1B (left lanes) is a 100 base pair DNA ladder (Gibco/BRL). CTGF mRNA was not observed in adult RPE cells (data not shown). These results demonstrated that RPE cells and CECs, which are involved in the pathogenesis of many ocular disorders, including the development of PVR and CNV, express CTGF mRNA. Therefore, various ocular cells are capable of expressing CTGF mRNA.

### Example 2: RPE cells and CECs express CTGF polypeptide

The expression of CTGF polypeptide by RPE cells and CECs was investigated. RPE cells and CECs were isolated as described above in Example 1 and cultured in 6-well tissue culture plates. Cultured RPE cells were starved for 24 hours in serum-free DMEM. Cultured CECs were starved for 24 hours in serum-free Essential Basal Medium (EBM) (BioWhittaker, Walkersville, MD), containing 0.1% bovine serum albumin, for 24 hours. Serum-free RPE cell media was replaced with fresh serum-free media containing transforming growth factor beta2 (TGFβ2) (1 to 30 ng/ml) or vascular endothelial growth factor (VEGF) (10 to 50 ng/ml). Serum-free CEC media was replaced with fresh media containing 1% fetal bovine serum containing recombinant VEGF (10 to 50 ng/ml) or TGFβ2 (1 to 30 ng/ml). Both cell types were incubated for an additional 48 hours in the presence of growth factor. Cell lysates were collected and electrophoresed on TRIS-HCl 10% polyacrylamide gels (Ready Gel, Bio-Rad Laboratories, Hercules, CA) and the proteins were transferred from the gels to PVDF blotting membranes (Millipore, Bedford, MA). The presence of CTGF in cell lysates was examined by western blot analysis. The membranes were probed for CTGF using a polyclonal rabbit anti-CTGF antibody, prepared using standard methods known in the art, followed by HRP-conjugated goat anti-rabbit secondary antibody (Vector Laboratories, Burlingame, CA), using standard methods. Image development was performed with the addition of ECL chemiluminescent detection solution (Amersham Pharmacia Biotech, Cleveland, OH) and exposure to Hyperfilm (Amersham Pharmacia Biotech).

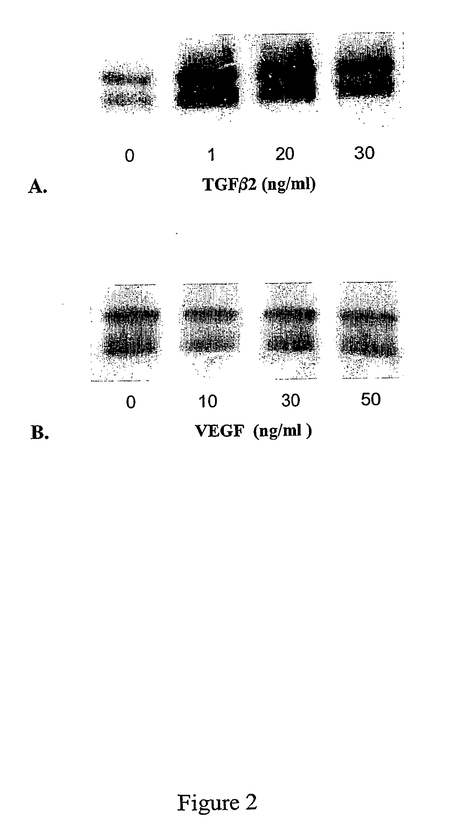

Cultured RPE cells expressed CTGF polypeptide. As the western blots in Figure 2A and Figure 2B show, in the absence of TGFβ2 or VEGF addition, detectable levels of CTGF protein were observed in untreated RPE cell lysates. Stimulation of RPE cells with various concentrations of TGFβ2 (1 to 30 ng/ml) for 48 hours in serum-free media resulted in increased CTGF protein expression compared to non-treated control cells. (Figure 2A.) The stimulation of CTGF expression in RPE cells by TGFβ2 was dose-dependent, as shown in Figure 2A. Increased expression of CTGF (above that of control) was observed in RPE cells treated with TGFβ2 concentrations as low as 1 ng/ml. These results showed that RPE cells synthesized CTGF polypeptides, and that CTGF expression by RPE cells was up-regulated by TGFβ2 in a dose-dependant manner. No increase in CTGF expression was detected in RPE cells treated with VEGF was detected. (Figure 2B.)

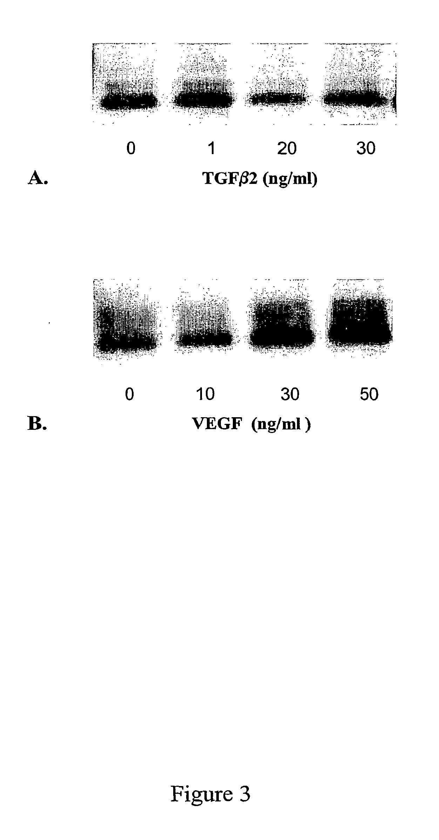

Cultured CECs were also found to express CTGF protein. The western blots in Figure 3A and Figure 3B show that detectable levels of CTGF protein were observed in lysates from untreated CECs. Stimulation of CECs with various concentrations of VEGF (10 to 50 ng/ml) for 48 hours in low-serum media resulted in increased CTGF protein expression compared to non-treated control cells. Stimulation of CTGF expression in CECs by VEGF was dose-dependent, as shown in Figure 3B. No increase in CTGF expression was detected in CECs treated with TGFβ2. (Figure 3A.) Taken together, these results demonstrated that RPE cells and CECs, which have important roles in the pathogenesis of many ocular disorders, such as PVR and CNV, express CTGF polypeptides and that CTGF expression in these cells is differentially regulated by TGFβ2 and VEGF.

In addition to showing that ocular cells expressed CTGF protein, these results also demonstrated that CTGF expression in ocular cells is regulated by various growth factors and cytokines which are located within the eye. Additionally, these results showed that differential regulation of CTGF expression occurred in various ocular cells. Specifically, TGFβ2 stimulated increased expression of CTGF in RPE cells. Within the eye, vitreous contains high levels of TGFβ. (Limb et al. (1991) Eye 5:686-693.) Various ocular disorders are associated with RPE cell exposure to vitreous. Therefore, the initiation, progression, or maintenance of various ocular disorders may result from TGFβ stimulation of CTGF expression in ocular cells, such as RPE. Increased expression of VEGF in ocular neovascularization has been reported. (Lopez et al. (1996) Invest Ophthalmol Vis Sci 37:855-868; Simo et al. (2002) Am J Ophthalmol 134: 376-382.) Therefore, various ocular disorders associated with increased expression of VEGF show increased CTGF when choroidal or retinal endothelial are exposed to VEGF.

CTGF levels in the supernatants of cultured RPE cells were determined by ELISA analysis. Cultured human RPE cells were treated with or without TGFβ2 (20 ng/ml) for 48 hours, as described above. Cell supernatants were harvested and cleared by centrifugation at 5,000 rpm for 5 minutes. Samples were stored at -70°C until ELISA assays were performed. ELISA data indicated that non-treated control RPE cells secreted CTGF (9 ng/ml). RPE cells treated with TGFβ2 secreted increased levels of CTGF (47 ng/ml). Taken together, these results indicated that ocular cells synthesize and secrete CTGF. Additionally, these results showed that CTGF synthesized by ocular cells can be measured qualitatively and quantitatively.

### Example 3: Production of recombinant human CTGF

Recombinant human CTGF (rhCTGF) was prepared as follows. A full-length human CTGF cDNA (DB60R32) was obtained from Dr. Gary Grotendorst (University of Miami Medical School). (Bradham et al. (1991) J Cell Biol 114:1285-1294.) A CTGF cDNA comprising only the open reading frame was generated by the polymerase chain reaction using DB60R32 DNA as template and the following primers (5'-gctccgcccgcagtgggatccatgaccgccgcc-3' (SEQ ID NO:5) and 5'-ggatccggatcctcatgccatgtctccgta-3') (SEQ ID NO:6), which added BamHI restriction enzyme sites (underlined) to either end of the amplified product. The resulting amplified DNA fragment was digested with BamHI, purified on an agarose gel, and subcloned directly into the BamHI site of the baculovirus (donor) expression plasmid pFastBacl (Invitrogen, Carlsbad, CA). The pFastBac1/CTGF cDNA vector construct was transposed into bacmid DNA and recombinant baculovirus was generated by following the manufacturer's protocol outlined in the BAC-TO-BAC Baculovirus Expression System manual (Invitrogen). Expansion of recombinant baculovirus titers in Sf9 insect cells was performed using standard procedures known in the art. (Murphy and Piwnica-Worms, (1984) Current Protocols in Molecular Biology, Vol. 2 (Ausubel et al., Eds.) John Wiley & Sons, Inc.)

Expression and production of rhCTGF was performed as follows.
HIGH FIVE insect cells, adapted to suspension growth, were grown in SF900II medium (Invitrogen) to a cell density of 1.0x10⁶ cells/ml. The cells were then infected with baculovirus containing CTGF at a multiplicity of infection of 10:1. Following infection, the cells were incubated at 27°C for 40 hours. The cells were then pelleted by centrifugation, and the CTGF-containing conditioned medium was collected and filtered through a 0.22 µm filter. The conditioned medium was then directly applied to a 5 ml Hi-Trap heparin column (Amersham Biosciences Corp., Piscataway NJ), which had been pre-equilibrated with 50 mM Tris, pH 7.2. The heparin column was then washed with 100 ml of 350 mM NaCl/50 mM Tris, pH 7.2, and bound rhCTGF was eluted with a linear gradient of 350 mM NaCl to 1200 mM NaCl over 15 column volumes. Fractions were analyzed for the presence of rhCTGF by SDS-PAGE.

Fractions containing rhCTGF were pooled and diluted 1:4 with 50 mM Tris, pH 7.5. The rhCTGF pool was loaded over a carboxy methyl (CM) ion exchange column (POROS CM column, PerSeptive Biosystems, Framingham, MA), which had been pre-equilibrated with 10 column volumes of 50 mM Tris, pH 7.5. The CM column was washed with 350 mM NaCl/50 mM Tris, pH 7.5, and bound rhCTGF was eluted with a linear gradient of 350 mM NaCl to 1200 mM NaCl over 15 column volumes. The fractions were analyzed for the presence of CTGF by SDS-PAGE. Fractions containing rhCTGF were pooled and used as rhCTGF material.

### Example 4: Production of rhCTGF N-terminal and C-terminal fragments

N-terminal fragments and C-terminal fragments of rhCTGF were prepared as follows. Recombinant human CTGF, prepared and purified as described above, was digested by treatment with chymotrypsin beads (Sigma Chemical Co., St. Louis MO), using 1.5 mg of rhCTGF per unit of chymotrypsin. Chymotrypsin treatment of rhCTGF was allowed to proceed at room temperature for 6 hours. The digested product and chymotrypsin beads were centrifuged, the chymotrypsin beads were discarded, and the supernatant, containing enyzmatically cleaved rhCTGF, was diluted 1:5 with 50 mM Tris, pH 7.5. The diluted supernatant was applied to a Hi-Trap heparin column. The flow-through was collected, and contained N-terminal fragments of rhCTGF. The heparin column was washed with 350 mM NaCl, and bound C-terminal fragments of rhCTGF and undigested rhCTGF were eluted with a linear gradient of 350 mM to 1200 mM NaCl, as described above. The fractions were analyzed for the presence of C-terminal fragments of rhCTGF by SDS-PAGE. Fractions containing C-terminal fragments of rhCTGF were pooled according to the observed purity of C-terminal rhCTGF.

The heparin column flow-through, which contained N-terminal fragments of rhCTGF, was adjusted to contain 0.5 M ammonium sulfate /50 mM Tris, pH 7.5. The sample containing N-terminal fragments of rhCTGF was then loaded onto a 15 ml phenyl sepharose HP column (Amersham Pharmacia Biotech), which had been pre-equilibrated with 0.5 M ammonium sulfate /50 mM Tris, pH 7.5. The phenyl sepharose HP column was then washed with 15 column volumes of 0.5 M ammonium sulfate/ 50 mM Tris, pH 7.5. Bound N-terminal fragments of rhCTGF were eluted with a linear gradient of 0.5 M to 0.0 M ammonium sulfate /50 mM Tris, pH 7.5, over approximately 15 column volumes. Fractions were analyzed for the presence of N-terminal fragments of rhCTGF by SDS-PAGE. Fractions containing N-terminal fragments of rhCTGF were pooled. This pool, containing N-terminal fragments of rhCTGF, was concentrated and the buffer exchanged with 50 mM Tris, 400 mM NaCl, pH 7.2, using Amicon ultrafiltration, YM10 membrane.

### Example 5: CTGF stimulates RPE cell and CEC attachment to fibronectin

Extracellular matrix molecules play a crucial role in mediating various activities of ocular cells, including ocular cell differentiation, attachment, migration, and proliferation. To examine the role of CTGF in extracellular matrix production in ocular cells and ocular disorders, the effect of CTGF on RPE cell and CEC attachment to fibronectin was investigated by performing the following assay.

RPE cells or CECs (1 x 10⁵ cells/ml), isolated and cultured as described above in Example 1, were removed from tissue culture plates by trypsinization. RPE cells were resuspended in cell culture media containing 0.4% fetal bovine serum, allowed to attach to the plate, and incubated with various concentrations ofrhCTGF (10 to 100 ng/ml) for 48 hours at 37°C. RPE cells were detached from the plate and 1 x 10⁴ cells (in 100 microlitres) were then added to 96-well tissue culture plates (Becton Dickinson Labware, Bedford, MA) previously coated with fibronectin (50 µg/ml). The cells were allowed to attach to the fibronectin-coated plates for 60 minutes at 37°C. The wells of the plates were then gently washed twice with phosphate-buffered saline (PBS) to remove non-attached cells. Fresh medium (150 µl) containing 20 µl of a 5 mg/ml solution of MTT (3-(4,5-dimethyl-2-thiazolyl)-2,5-diphenyl-2H tetrazolium bromide, Sigma Chemical Co.) was added to each well, and the plates, which contained attached cells, were incubated for 5 hours at 37°C. The supernatants were removed from the wells and formazan precipitates associated with attached cells were solubilized with 100 µl of 100% DMSO (Sigma Chemical Co.), followed by placement of the plates on a shaker for 10 minutes. Absorbance at 550 mm was determined using a Dynatech MR 600 microplate reader. This MTT assay provides a quantitative analysis of the measurement of the number of cells remaining attached to the fibronectin-coated tissue culture plates. This assay is performed following shaking of the plates, and therefore correlated with the strength of attachment of the cells to fibronectin. The number of attached cells was proportional to the absorbance measured in the MTT assay described.

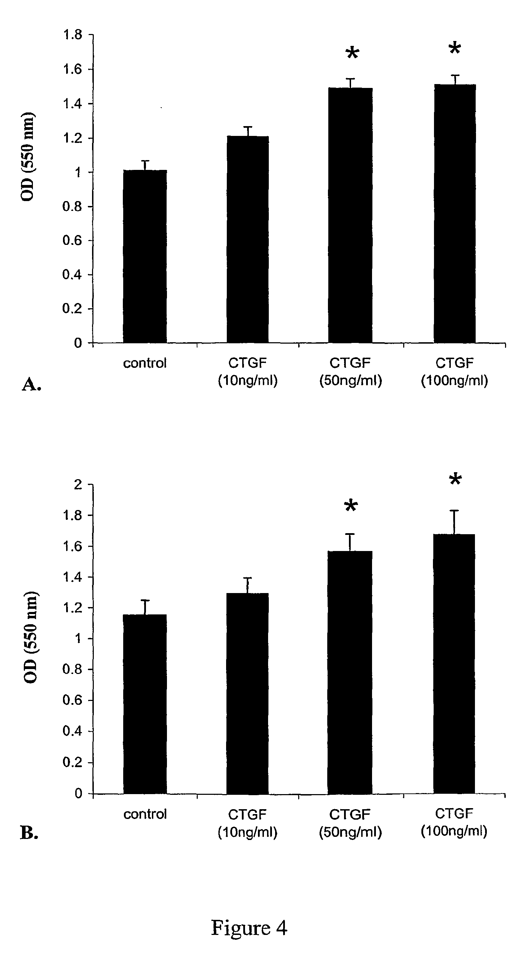

Both RPE cells (Figure 4A) and CECs (Figure 4B) showed increased attachment to fibronectin following treatment with rhCTGF compared to non-treated control cells. The effect of rhCTGF on RPE cell and CEC attachment to fibronectin was dose-dependant, as shown in Figure 4A and Figure 4B. In both RPE cells and CECs, a significant increase in cell attachment to fibronectin was observed in rhCTGF-treated cells, compared to control cells, at rhCTGF concentrations of 50 ng/ml or 100 ng/ml (indicated by asterisks (P<0.05) in Figure 4A and Figure 4B). These results indicated CTGF affects extracellular matrix production in ocular cells, at least in part by stimulating the production of fibronectin in RPE cells and CECs.

### Example 6: CTGF simulates chemotaxis of RPE cells and CECs

Ocular cell migration and chemotaxis are associated with many ocular disorders, including choroidal neovascularization and PVR. The effect of CTGF on chemotaxis of RPE cells and CECs was investigated. To examine chemotaxis, cell migration assays were performed using a modified Boyden chamber assay as previously described. (He et al. (1998) Biochem Biophys Res Commun 249:253-257.) RPE cells and CECs were isolated as described above in Example 1, and cultured on fibronectin-coated inserts (50 µg/ml) of 24-well Boyden chamber plates in media containing 0.4% fetal bovine serum. Various concentrations of rhCTGF (1 to 50 ng/ml) were added to the lower wells of the essay plates to examine the chemotactic activity of CTGF for RPE cells and CECs. The cells were then incubated for 5 hours, after which the chamber inserts were washed three times with PBS, fixed with ice-cold 100% methanol for 10 minutes, and counterstained with hematoxylin for 20 minutes. The number of cells that migrated was counted using phase-contrast microscopy (320x). The number of cells that migrated was determined in four randomly chosen fields per insert.

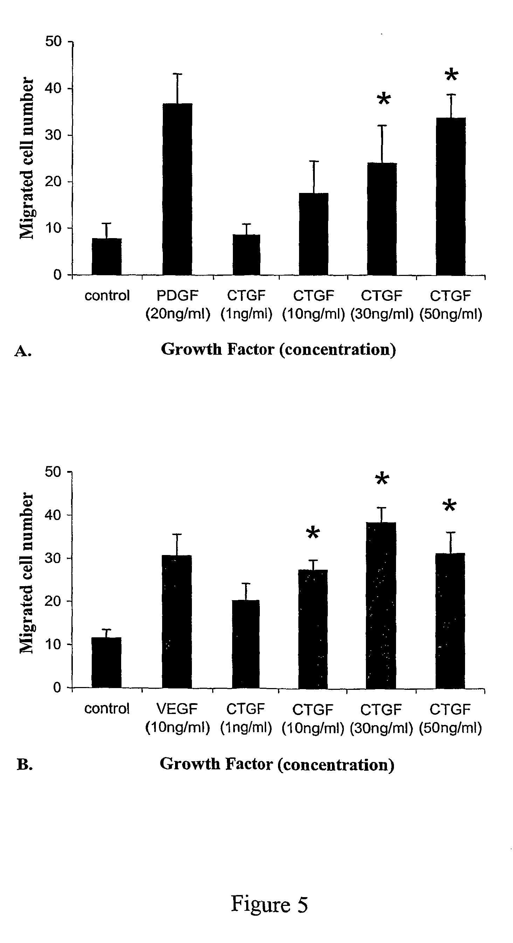

Figure 5A and Figure 5B show that rhCTGF stimulated migration and chemotaxis of RPE cells and CECs in a dose-dependant manner. As shown in Figure 5A, a significant increase in RPE cell migration above control occurred following addition of either 30 ng/ml or 50 ng/ml rhCTGF (indicated by asterisks (P<0.01) in Figure 5A). With the addition of 50 ng/ml rhCTGF, the extent of RPE cell migration was similar to that observed with platelet derived growth factor (PDGF) (20 ng/ml), a potent chemoattractant factor for RPE cells, which served as a positive chemoattractant factor control. Therefore, rhCTGF displayed potent chemotactant activity to RPE cells, and was equally effective at stimulating chemotaxis as PDGF, a known potent chemoattractant factor.

As shown in Figure 5B, rhCTGF stimulated the migration and chemotaxis of CECs above that observed in non-treated control cells. Addition of rhCTGF at concentrations of 10 ng/ml, 30 ng/ml, and 50 ng/ml resulted in significant increases in CEC migration above non-treated controls (indicated by asterisks (P<0.05) in Figure 5B). Addition of rhCTGF at concentrations of 30 ng/ml and 50 ng/ml were at least as effective at stimulating CEC migration as was VEGF (10 ng/ml), a known potent chemoattractant factor to CECs. Therefore, rhCTGF displayed potent chemotactant activity to CECs, and was equally as effective at stimulating chemotaxis in CECs as VEGF, a known potent chemotactic factor. Taken together, these results indicated CTGF was a potent chemotactic factor for various ocular cells.

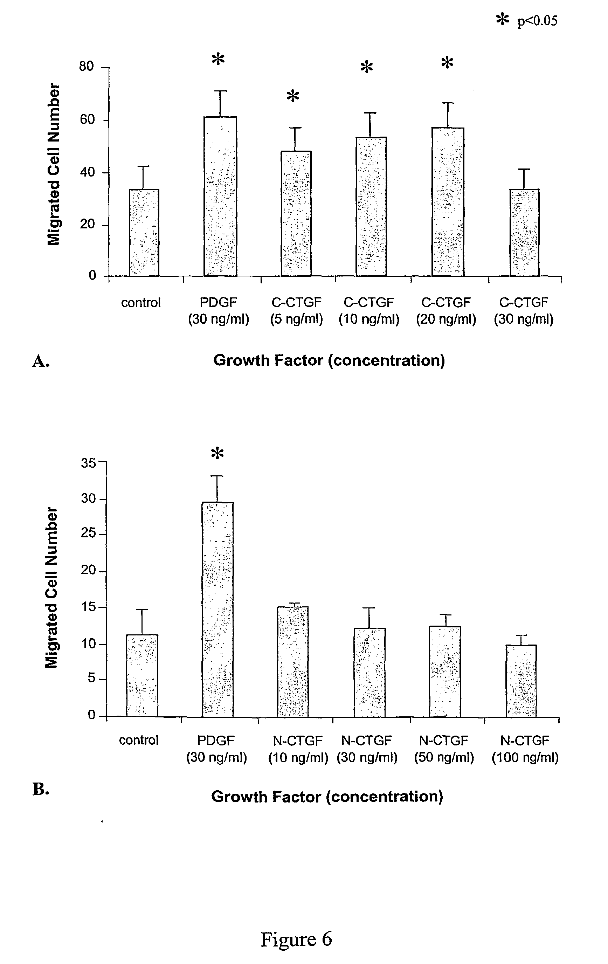

The effect of CTGF fragments on chemotaxis of RPE cells was also investigated. Various concentrations of recombinant human CTGF N-terminal fragment (10 to 100 ng/ml) and C-terminal fragment (5 to 30 ng/ml) were tested in the cell migration assay as described above. As shown in Figure 6A, rhCTGF C-terminal fragments stimulated migration and chemotaxis of RPE cells in a dose-dependent manner. A maximal chemotactic effect of CTGF fragments in RPE cells was observed upon addition of 20 ng/ml rhCTGF C-terminal fragment. With the addition of 20 ng/ml rhCTGF C-terminal fragment, the extent of RPE cell migration was similar to that observed with PDGF. Additionally, the chemotactic response observed in RPE cells at 20 ng/ml C-terminal fragment of rhCTGF was essentially equivalent to that seen using 30 ng/ml rhCTGF (see this Example above, and Figure 5A). Only a minor increase in cell migration above control was observed in RPE cells treating with N-terminal fragment of CTGF. (Figure 6B.)

Together, these results showed that CTGF and fragments of CTGF stimulated ocular cell chemotaxis and migration. Therefore, CTGF and fragments of CTGF provide novel therapeutic targets for inhibiting ocular cell migration associated with various ocular disorders.

### Example 7: CTGF stimulates RPE cell proliferation

Under normal conditions in the healthy eye, RPE cells are quiescent, and therefore do not proliferate. In many ocular disorders, however, ocular cell proliferation, including RPE cell proliferation, is observed and associated with development and progression of various ocular disorders. Therefore, the effect of CTGF on ocular cell proliferation was examined as follows.

RPE cell proliferation was measured using a ³H-thymidine uptake/incorporation assay. Human RPE cells were isolated as described above in Example 1 and incubated in 24-well tissue culture plates for 48 hours in DMEM containing 1% fetal bovine serum, in the presence or absence of various concentrations of rhCTGF (1 to 100 ng/ml). Afterward, the RPE cell cultures were pulse-labeled with ³H-thymidine (1 µCi/well, Amersham) for the final 16 hours of incubation. The amount of ³H-thymidine uptake in the cultures was then determined as previously described. (He et al., *supra*.)

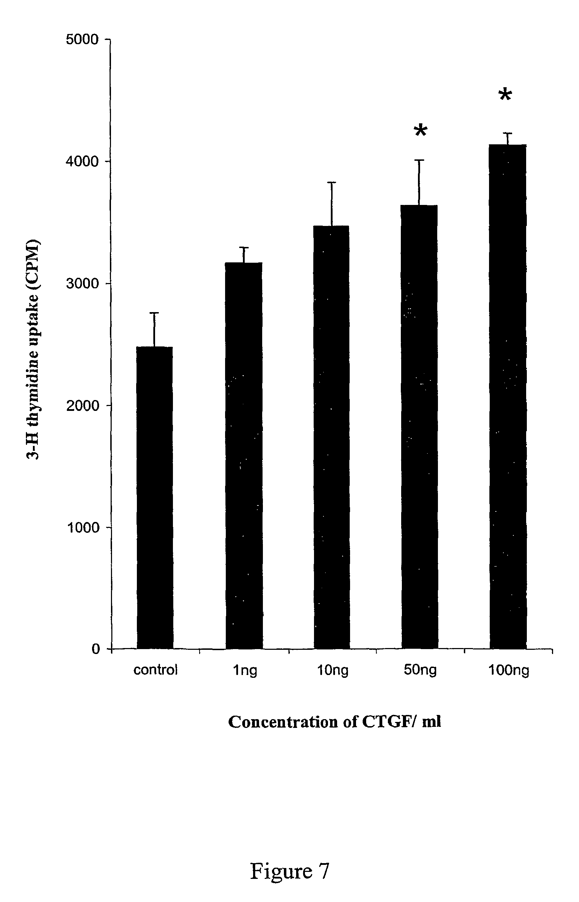

Figure 7 shows rhCTGF displayed mitogenic activity to RPE cells. Specifically, rhCTGF stimulated a dose-dependent increase in ³H-thymidine incorporation in RPE cells after 48 hours of rhCTGF treatment. At rhCTGF concentrations of 50 ng/ml and 100 ng/ml, rhCTGF stimulated an approximately 20% increase in ³H-thymidine uptake in RPE cells, a statistically significant increase above that of control (indicated by asterisks (P<0.05) in Figure 7). These results demonstrated RPE cell proliferation was induced by CTGF. Therefore, CTGF stimulates proliferation of ocular cells.

### Example 8: CTGF induction of CEC tube formation

Angiogenesis and neovascularization involve a complex and highly orchestrated series of cellular events and changes in endothelial cell function, including proliferation, migration, and differentiation. To assay the effect of CTGF on endothelial cell function and neovascularization in the eye, the following tube formation assay was performed on CECs. The tube formation assay is a complex three-dimensional assay that examines the ability of endothelial cells to form primitive vascular structures or vascular tubes in culture. For primitive vascular tubes to form using this assay, endothelial cells undergo proliferation, migration, differentiation, and organization into three-dimensional structures. Therefore, the effect of CTGF on CEC tube formation was examined using this assay.

Bovine CECs were isolated as described above in Example 1. Monolayer cultures of CECs were grown on fibronectin-coated 6-well plates. Growth media was removed and replaced with fresh EGM medium containing 1% fetal bovine serum. Recombinant human CTGF (20 ng/ml) was added to the cultures and the CECs were incubated for an additional 9 days. Tube formation was monitored and photographed daily using phase-contrast microscopy.

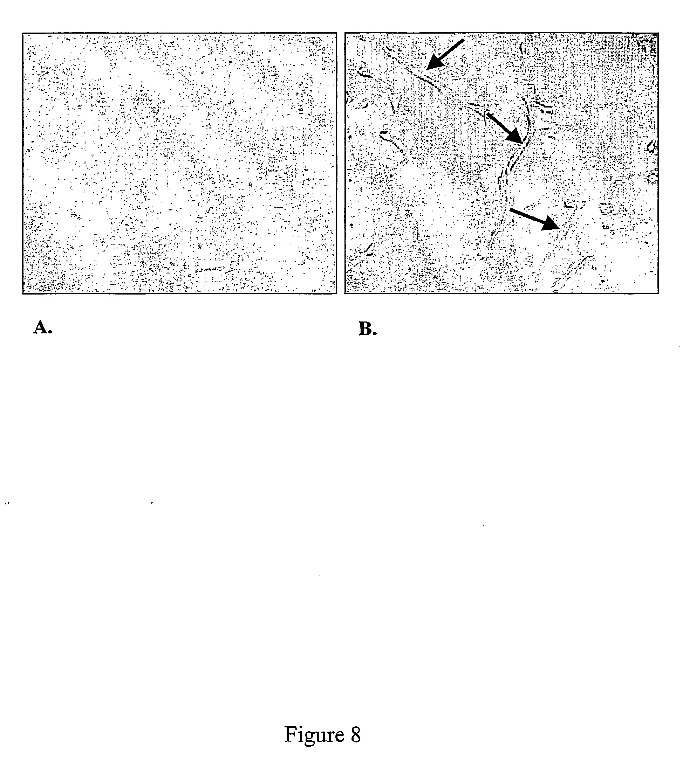

Monolayer cultures of CECs showed increased tube formation in response to rhCTGF addition. As shown in Figure 8A, in the absence of rhCTGF addition, CECs remained as monolayer cultures. As shown in Figure 8B, in the presence of rhCTGF, the attached endothelial cells became extended, formed cell-cell contacts, and established a branched network of tube-like structures. (See arrows, Figure 8B.) These results were similar to those previously reported, showing the effect of VEGF (a known angiogenic factor) on endothelial cell tube formation. (Murata et al. (2000) Invest Ophthalmol Vis Sci 41:2309-2317.) Therefore, these results demonstrated rhCTGF induced tube formation in cultured CECs, indicating CTGF stimulated ocular endothelial cell differentiation, proliferation, and migration. Additionally, these results suggested CTGF could induce neovascularization in the eye, including neovascularization associated with various ocular disorders.

### Example 9: CTGF induces expression of extra domain A fibronectin in RPE cells

Increased expression of extra domain A fibronectin (EDA(+)FN) is associated with tissue injury, including ocular tissue injury. Specifically, cellular fibronectin expression has been identified in tissue samples obtained from patients with PVR and CNV. To examine the effect of CTGF on cellular fibronectin expression in ocular cells, the following experiments were performed.

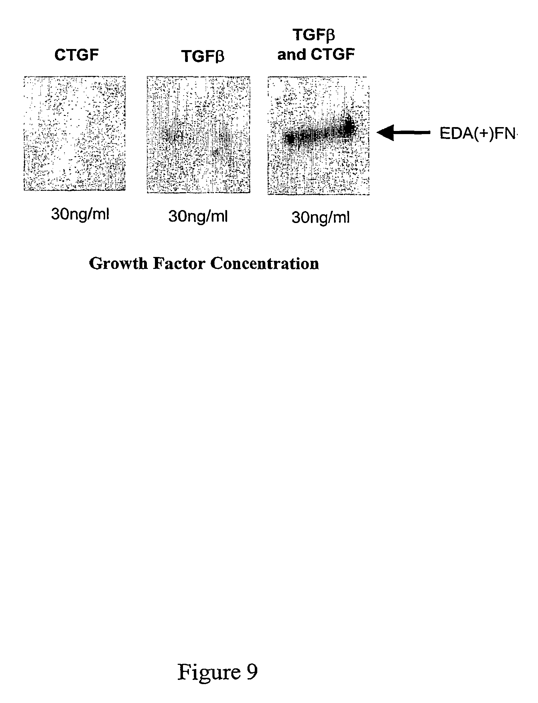

Cultures of human fetal RPE cells (passages 2 to 4) were grown to subconfluence as described in Example 1. RPE cells were serum starved for 24 hours followed by treatment with rhCTGF (30 ng/ml), TGFβ2 (30 ng/ml), or both growth factors, for 48 hours in serum-free medium. Afterward, RPE cells were collected and lysed using lysis buffer. The resulting cellular homogenates were passed through a 25G needle to sheer DNA, incubated on ice for 30 minutes, and then centrifuged at 12,000 rpm for 10 minutes at 4°C. Samples were examined for cellular fibronectin expression (EDA(+)FN isoform) by western blot analysis using a FN EDA-specific monoclonal antibody (Clone IST-9; Accurate Chemicals, Westbury, NY). As shown in Figure 9, addition of rhCTGF alone resulted in no detectable EDA(+)FN expression in RPE cells. Addition of TGFβ2 alone resulted in weak EDA(+)FN expression in RPE cells. When both CTGF and TGFβ2 were added to cultured RPE cells, a robust increase in EDA(+)FN expression was observed. These results demonstrated that CTGF enhances the ability of TGFβ to stimulate extracellular matrix production in ocular cells.

### Example 10: Fibronectin induces expression of CTGF in RPE cells

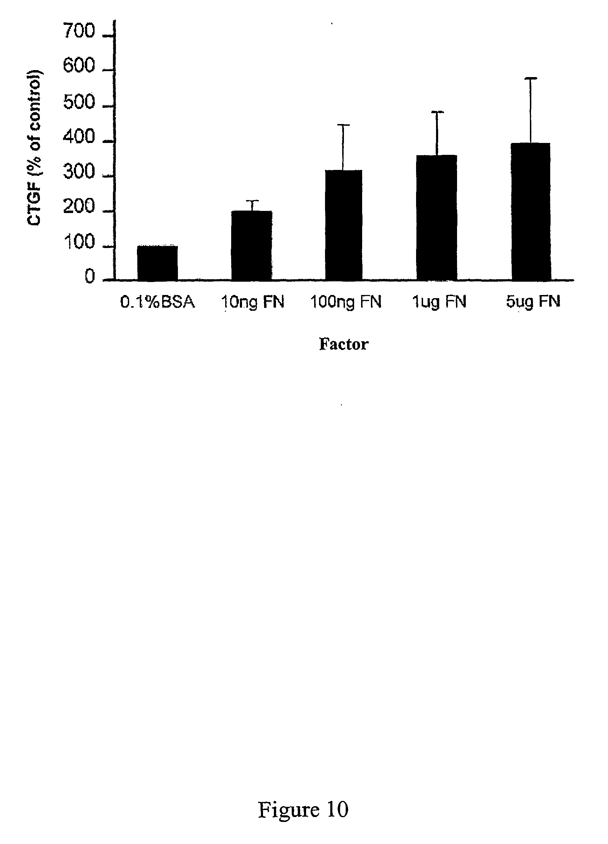

Fibronectin is abundantly expressed in PVR and conditions associated with retinal or choroidal neovascularization. To examine the effects of CTGF on fibronectin expression in ocular cells, the following experiment was performed. RPE cells, isolated as described above in Example 1, were seeded on plastic in 6-well plates pre-coated with various amounts of fibronectin (10 ng to 5 µg). After 24 hours, supernatants were tested for CTGF expression using an ELISA. Cellular homogenates were tested for CTGF expression using western blot analysis. As shown in Figure 10, RPE cells plated on fibronectin showed a dose-dependent increase in CTGF expression, as measured by densitometric analysis of the western blot analysis of cell homogenates. RPE cells plated on as little as 10 ng fibronectin demonstrated increased CTGF expression above that observed in control RPE cells plated on 0.1% BSA. Fibronectin induced an 80% increase above control in CTGF expression in cellular homogenates of RPE cells plated on fibronectin. ELISA analysis revealed a doubling of the level of both CTGF and N-terminal fragments of CTGF in the supernatants of these cells (data not shown). These results provided evidence that fibronectin stimulates CTGF expression in RPE cells as RPE cells migrate into various ocular environments containing fibronectin, which occurs in various ocular disorders.

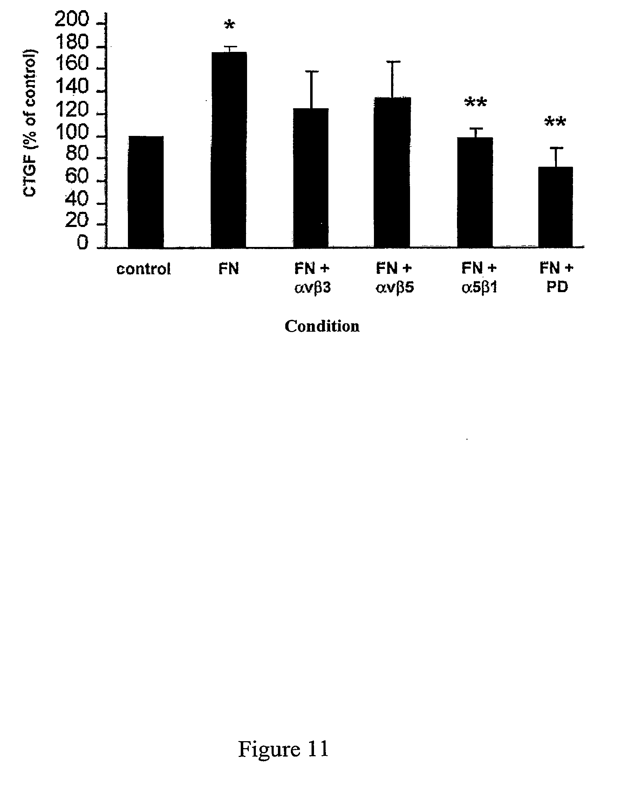

In parallel experiments, pretreatment of cells with neutralizing antibodies directed against α₅β₁ integrin, A fibronectin-specific integrin, significantly blocked the increase in CTGF expression observed in both cell homogenates and supernatants. (Figure 11.) Addition of antibodies directed against αᵥβ₃ integrin and αᵥβ₁ integrin had no significant effect on fibronectin-induced CTGF expression. These results further supported a role of fibronectin in stimulating CTGF production in ocular cells, as α₅β₁ integrin is associated with attachment of RPE cells to fibronectin.

### Example 11: CTGF expression and its effect on retinal Müller cell migration

Rat retinal Müller cells were obtained from an established cell line (Sarthy et al. (1998) Invest Ophthalmol Vis Sci 39(1):212-6), and were cultured in the same medium as the RPE cell culture described in Example 1. Expression of CTGF mRNA was examined in cultured retinal Müller cells using RT-PCR. The methods for isolation mRNA and cDNA synthesis from Müller cell were the same as RPE (see example one for detail). PCR amplification was performed for detection of CTGF mRNA expression in the cell using oligonucleotide sequence primer specific for rat CTGF. The primer sequences for rat CTGF were 5'-ctccagtctgcagaaggtattc-3' (SEQ ID NO.:7) and 5'-caaccgcaagattggagtgt-3' (SEQ ID NO.:8), which resulted in amplification of a 480 base pair rat CTGF DNA fragment. The conditions used for PCR amplification were as follows: 94°C for 1minute; 60°C for 1 minute; and extension at 72°C for 2 minutes, total for 35 cycles. The PCR product of rat retinal Müller cell CTGF was resolved in an agarose gel. A PCR amplification product of the expected size was observed (data not shown), indicating rat retinal Müller cells express CTGF.

Müller cell migration was assayed by using a modified Boyden chamber assay, as describd above in Example 6. CTGF induced Müller cell migration in dose dependent manner. At a dose of 30ng/ml and 50ng/ml, rhCTGF stimulated a significant increase of migration of Müller cells on fibronectin (data not shown). These results showed CTGF was a chemotactic for Müller cells. These results indicated a role of CTGF on Müller cell migration in development of PVR, as Müller cells are present in PVR membrane.

### Example 12: CTGF expression in human PVR membrane tissue

The expression of CTGF in human PVR membranes obtained from individuals with ocular disorders was examined by immunohistochemical analysis. PVR membranes, formed as a result of trauma to the eye or retinal detachment, were surgically removed from patients. The isolated PVR membranes were prepared for immunostaining as follows. Isolated PVR membranes were snap-frozen and sectioned at 6 µm using a cryostat. Thawed tissue sections were air-dried, rehydrated with PBS (pH 7.4), and blocked with 5% normal goat serum for 15 minutes. Sections were incubated with rabbit anti-CTGF polyclonal antibody (primary antibody, prepared using standard methods known in the art) for 60 minutes. Sections were then washed three times with PBS, 5 minutes each wash, to remove unbound anti-CTGF antibody. Sections were subsequently incubated with biotinylated anti-rabbit antibody (secondary detection antibody, diluted 1:400, Vector, Burlingame, CA), and washed for 5 minutes with PBS. Streptavidin peroxidase was added, and color development performed using AEC kit (Zymed). Slides were counter-stained with hematoxylin and mounted with glycerin-gelatin medium.

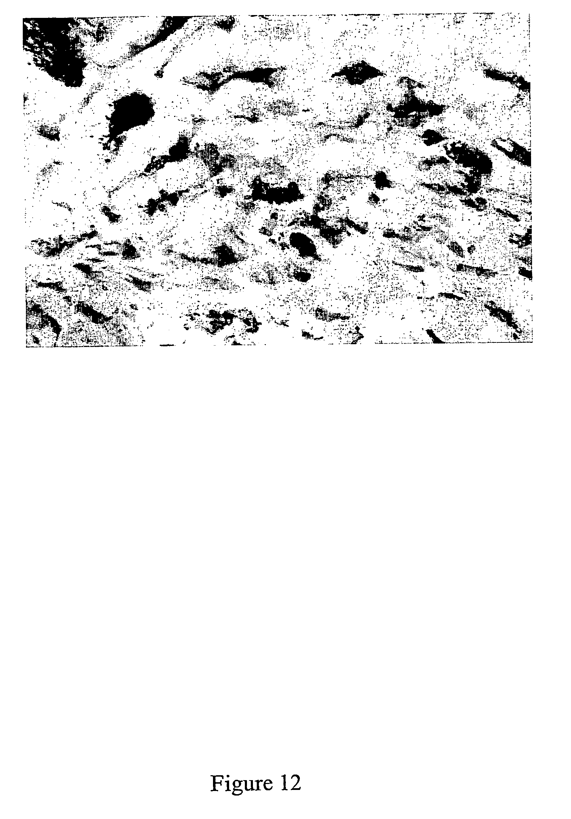

All ten isolated PVR membranes examined stained positive for CTGF expression, as evidenced by immunohistochemistry. Within PVR membranes, CTGF was localized to the extracellular space or to cells within the fibrotic PVR membranes, as shown in Figure 12. Immunostaining for CTGF was not detected in sections of normal, non-diseased RPE (data not shown). These results showed CTGF was expressed in ocular disorders.

Double immunoperoxidase and alkaline peroxidase staining was also performed on sections of isolated human PVR membranes. Thawed cryosections were fixed for 10 minutes with 10% neutral buffered formalin (Polyscience, Inc., Warrington, PA), washed three times (5 minutes each wash) with Tris-HCl (pH 7.4), and blocked with 5% goat serum for 15 minutes. After the sections were immunostained for CTGF using an anti-CTGF antibody, as described above, the slides were washed three times with Tris-HCl buffer. Primary antibody specific for cytokeratin (in the retina, cytokeratin is a specific marker for RPE cells), and primary antibody specific for CD-31 (an endothelial cell-specific marker) were added, and the slides were incubated for 1 hour at room temperature. The slides were washed three times with Tris-HCl buffer. The sections were incubated with alkaline phosphatase-conjugated anti-mouse antibody (Vector, diluted 1:500) for 30 minutes. The slides were again washed three times with Tris-HCl buffer. Vector Blue was then added to the slides for 15 minutes for color development.

CTGF was expressed in isolated human PVR membranes, as evidenced by immunohistochemical analysis. (Figure 12.) Double-labeling of isolated human PVR membrane sections revealed that many CTGF-positive cells also stained positive for cytokeratin, an RPE cell-specific marker in the retina. These results showed that RPE cells within PVR membranes expressed CTGF.

### Example 13: CTGF expression in CNV membranes

The expression of CTGF in CNV membranes was examined by immunohistochemical staining. CNV membranes were surgically excised from patients with clinically diagnosed age-related macular degeneration. The isolated CNV membranes were prepared for analysis of CTGF expression by immunostaining using methods described in Example 12 above for CTGF immunostaining of PVR membranes.

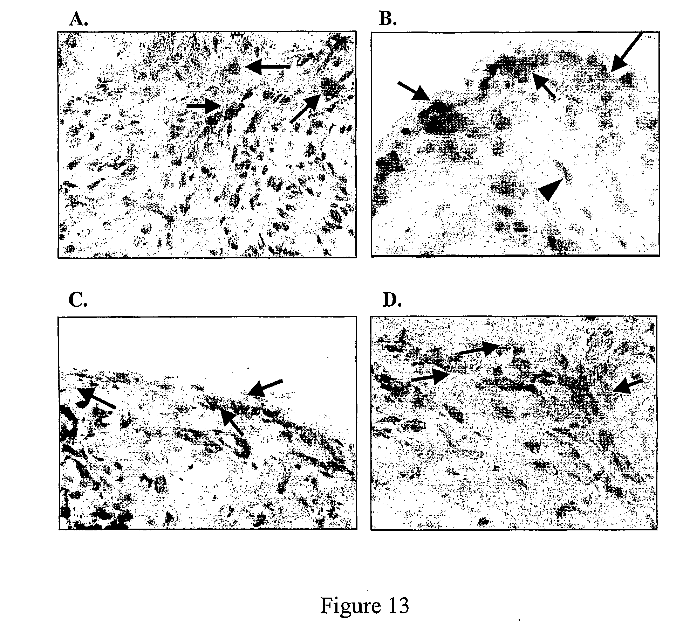

CNV membranes examined stained positive for CTGF expression. The most prominent CTGF staining occurred in vascularized regions of the membrane, as indicated with arrows in Figure 13A. Figure 13A shows cytoplasmic staining of CTGF-positive cells (indicated by arrows). The nuclei of cells are stained with hemotoxylin. In various ocular disorders, certain membranes show active neovascularization, while others display fibrous scars with little neovascularization. The results indicated that in CNV, CTGF was predominantly localized to neovascular membranes; however, some CTGF staining was also found in non-neovascular membranes. The intensity and extent of CTGF staining within CNV membranes was several fold greater in vascular areas compared to that observed in non-vascular membranes and fibrotic membranes. Additionally, CTGF-positive cells were found surrounding blood vessels in CNV membranes.

A partially intact RPE monolayer was observed in certain isolated CNV membranes. As indicated by arrows in Figure 13B, the regions associated with an RPE monolayer stained strongly positive for CTGF. Double staining of the CTGF-positive cells within the intact RPE monolayer demonstrated that many CTGF-positive cells were also positive for cytokeratin immunoreactivity, indicated by arrows in Figure 13C. The arrowhead shown in Figure 13B shows a cell migrating form the RPE monolayer stained positive for CTGF. These results indicated that the CTGF-positive cells observed in isolated CNV membranes swere transdifferentiated RPE cells, displaying a change in morphology from that of a typical retinal pigment epithelial cell to a more fibroblast-like appearance. Arrows in Figure 13D show other CTGF-positive cells in CVN membranes included endothelial cells, as evidenced by double-labeling with the endothelial cell marker CD-31 and CTGF. Normal adult retinas were also examined for CTGF expression; however, no CTGF immunoreactivity was detected in RPE or CEC of these samples (date not shown).

Taken together, these results demonstrated that CTGF is expressed in CNV membranes associated with ocular disorders. CTGF expression in CNV membranes was most prominent in vascular regions of the membranes. Expression of CTGF was localized to stromal RPE and CEC as well as RPE in the residual monolayer. The presence of CTGF in the residual RPE monolayer suggested that CTGF could activate the normal underlying endothelium, leading to stimulation of choroidal angiogenesis and neovascularization, in part, by regulating CEC function.

### Example 14: CTGF expression in PVR

The expression of CTGF and fragments of CTGF in vitreous of patients clinically diagnosed with PVR was investigated. Vitreous samples were obtained from different patients with various ocular disorders, including retinal detachment with PVR (RD+PVR), retinal detachment without PVR (RD), epiretinal membrane (ERM), and macular hole (MH), which is not associated with PVR. ELISA assays specific for detecting CTGF and fragments of CTGF were used to determine the levels of CTGF and of fragments of CTGF in these vitreous fluids.

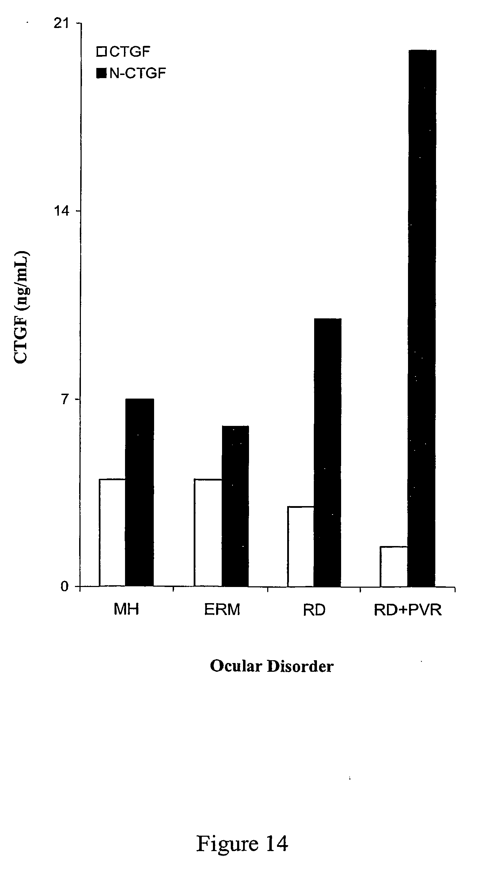

As shown in Figure 14, CTGF (open bars) and N-terminal fragments of CTGF (solid bars) were detected in vitreous fluid obtained from patients with various ocular disorders, including MH, ERM, RD, and RD+PVR. In all samples tested, detectable levels of N-terminal fragments of CTGF were higher than that of CTGF. A significantly higher level of N-terminal fragments of CTGF was observed in vitreous obtained from patients with PVR compared to that seen in vitreous obtained from patients with eye disease without PVR, such as MH. (Figure 14.) Specifically, the levels of N-terminal fragments of CTGF in vitreous of patients with RD was approximately 10 ng/ml, while the levels of N-terminal fragments of CTGF in vitreous of patients with RD complicated with PVR was approximately 20 ng/ml. (Figure 14.) Therefore, RD alone was not associated with a significant increase in the levels of N-terminal fragments of CTGF. However, in RD associated with PVR, N-terminal fragments of CTGF were elevated, suggesting a role of CTGF in the development of PVR in RD.

These results demonstrated that levels of CTGF and fragments of CTGF could be measured quantitatively from cell-free vitreous samples. These results also showed that CTGF levels are greatly elevated in vitreous of patients with ocular disease associated with fibrosis (fibrotic), such as PVR, and, to a lesser extent, in ocular disorders not having a fibrotic aspect, such as MH.

### Example 15: CTGF expression in CNV and retinal neovascularization

The expression of CTGF in vitreous of patients clinically diagnosed with various forms of ocular neovascularization was investigated. Vitreous samples were obtained from patients with clinically diagnosed CNV and retinal neovascularization (RNV). Samples representing CNV included age-related macular degeneration (AMD), and CNV without AMD (CNVM non-AMD). Samples representing RNV included proliferative diabetic retinopathy (PDR), PDR with vitreous hemorrhage (PDR with VH), and VH without PDR (VH non-PDR). ELISA assays specific for detecting CTGF and fragments of CTGF were used to determine the levels of CTGF and of fragments of CTGF in these vitreous fluids.

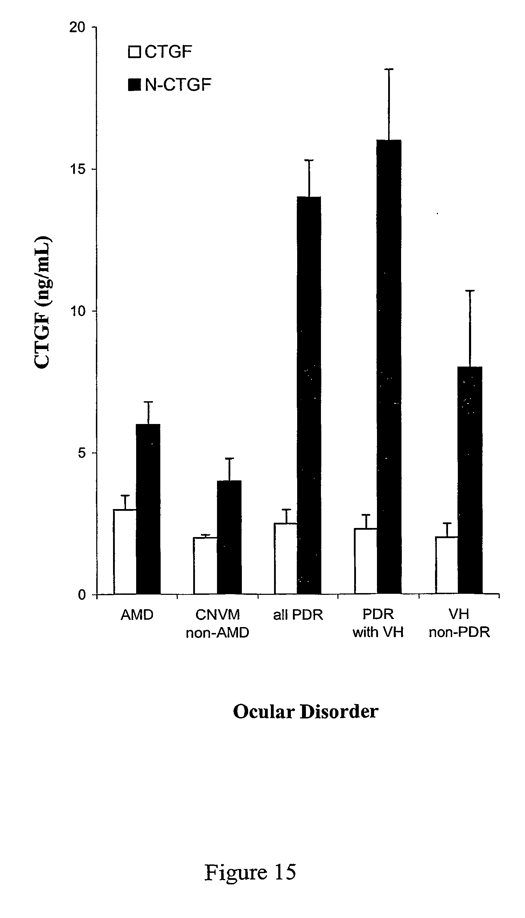

As shown in Figure 15, CTGF (open bars) and N-terminal fragments of CTGF (solid bars) were detected in vitreous fluid obtained from patients with various ocular disorders, including AMD, CNVM non-AMD, PDR, PDR with VH, and VH non-PDR. In all samples tested, detectable levels of N-terminal fragments of CTGF were higher than that of CTGF. CTGF N-terminal fragment levels were highest in vitreous samples obtained from patients diagnosed with PDR. These results showed that CTGF levels are greatly elevated in vitreous of patients with PDR. Additionally, these results indicated that CTGF expression is associated with ocular disorders associated with neovascularization and ocular cell proliferation.

### Example 16: Animal model of PVR involving RPE cell injection with PDGF

Experiments using animal models (pigmented rabbits) of ocular disease were performed. In these animal models of ocular disease, one eye (typically the right eye) is used experimentally from each animal, allowing the other eye to serve as a non-treatment control. Due to the inherent variability of *in vivo* animal model experiments, each experimental group typically involved 6 rabbits, allowing for the generation of sufficient numbers to achieve statistically valid results. In this established rabbit model of PVR, isolated RPE cells and PDGF are injected into the rabbit eye. In this model, 94% of eyes injected with RPE and PDGF developed retinal focal traction within one week following the injection, and 92% of the injected eyes developed retinal detachment within two weeks of injection.

Rabbit RPE cells were isolated from adult rabbit eyes (rabbits used in the studies weighed 2.5 kg to 3.5 kg.), and cultured in DMEM supplemented with 10% fetal bovine serum. Subconfluent cultures of rabbit RPE cells (typically at passage 2 to 3) were used for all subsequent injections. Cultured rabbit RPE cells were removed from culture plates by trypsinization and resuspended in PBS (250,000 cells per 0.1 ml). Prior to injection of RPE cells into rabbit eyes, 0.2 mls of aqueous humor was removed from each eye using a 25-guage needle. With indirect opthalmoscopic control, a 27-guage needle attached to a tuberculin syringe containing the RPE cells was passed through the sclera at a point three millimeters posterior to the limbus, at a position just over and above the optic disc. Following injection of RPE cells, 150 ng of PDGF-BB in 0.1 ml of PBS was injected through the same entrance site using the same technique as described for injection of RPE cells. Following the injections, animals were examined over time using indirect ophthalmoscopic examinations. The development and extent of PVR was monitored over time, and classified into one of five stages, based on clinical findings, according to parameters described by Fastenberg. (Fastenberg et al. (1982) Am J Ophthalmol 93:565-572.) The stages ofPVR development in this rabbit model are outlined in Table 1 below.

**TABLE 1**

| Stages of PVR in the rabbit eye | |
|---|---|
| Stage 0 | Normal Fundus |
| Stage 1 | Intravitreal membrane |
| Stage 2 | Focal traction |
| | Localized vascular change |
| | Hypermia, engorgement, dilation, tortuosity, vessel elevation |
| Stage 3 | Localized detachment of medullary ray |
| Stage 4 | Total ray detachment |
| | Peripapillary detachment |
| Stage 5 | Total retinal detachment |
| | Retinal holes |
| | Retinal folds |

Histological examination of the affected eyes in this animal model of PVR showed the presence of moderately cellular membranes with moderate fibrosis. When the number of RPE cells injected was reduced from 250,000 cells, as described above, to 100,000 cells, the resulting PVR was mild, and only fragile, paucicellular, non-fibrotic membranes developed. Additionally, membranes that developed in eyes injected with reduced numbers of RPE cells (i.e., 100,000 cells) were focal and localized, no retinal detachment was observed.

### Example 17: CTGF induces PVR in animal model of PVR

The effect of CTGF on the development of PVR was investigated, using a modification of the rabbit model of PVR described above in Example 16. Initially, various amounts of rhCTGF were injected into the rabbit eye. The effect of intra-vitreous rhCTGF injection alone on RPE, and any resulting development of PVR, was examined. As indicated in Table 2, vitreous injection of either 200 ng or 400 ng of rhCTGF into a healthy rabbit eye had no detectable effect on RPE. Subretinal injection of 200 ng of rhCTGF, however, resulted in minor RPE changes, including slight, focal disorganization of the RPE monolayer. No significant cell proliferation or membrane formation was observed. (See Table 2 below.)

**TABLE 2**

| Intravitreous injection of rhCTGF into the rabbit eye | | |
|---|---|---|
| Injection Site | Amount of rhCTGF Injected | Effect/Outcome |
| Vitreous injection | 200 ng CTGF | No effect on RPE |
| | 400 ng CTGF | No effect on RPE |
| Subretinal injection | 200 ng CTGF | Minor RPE changes |

In the rabbit model of PVR described above in Example 16, RPE cells injected into the rabbit eye, along with PDGF, resulted in development of a mild degree of PVR within two weeks of injection, characterized by formation of cellular membranes with moderate fibrosis. To examine the effects of CTGF in this rabbit model of mild PVR, RPE cells were injected into the rabbit eye, as described above, along with various amounts of rhCTGF, without PDGF injection. As shown in Table 3 below, when RPE cells were injected into the rabbit eye along with 200 ng rhCTGF, stage 1 PVR resulted after 3 weeks. Histological examination of the injected eye did not reveal the presence of epiretinal membranes or retinal detachment.

When RPE cells were injected into the rabbit eye along with 400 ng rhCTGF, grade 2 PVR developed after 3 weeks. Small and thin membranes developed, as determined by histological examination of the injected eye. The results of injecting various amounts of rhCTGF together with RPE cells into the rabbit eye are summarized in Table 3 below. Taken together, these results indicated that rhCTGF, when injected intravitreously, had a minimal effect on RPE.

**TABLE 3**

| Intravitreous injection of RPE cells and rhCTGF into the rabbit eye | | | |
|---|---|---|---|
| RPE Cells Injected | Amount of rhCTGF Injected | RPE Stage (after 3 weeks) | Histology |
| 100,000 RPE cells | No CTGF | Stage 0 | Negative |
| 100,000 RPE cells | 100 ng CTGF | Stage 0 | Negative |
| 100,000 RPE cells | 200 ng CTGF | Stage 1 | Negative |
| 100,000 RPE cells | 400 ng CTGF | Stage 2 | Small and thin membranes |

A positive control for the development of PVR in this animal model was intravitreous injection of 100,000 RPE cells with 50 ng PDGF. Three weeks following such injection, tractional retinal detachment was observed in the injected eye, associated with thin paucicellular membranes. (See Table 4 below.) In a parallel experiment, one week following the injection of 100,000 RPE cells with 50 ng PDGF, 200 ng rhCTGF was injected intravitreously. Two weeks later, eyes were examined for any changes. Under these experimental conditions, thick, fibrotic membranes developed, representing PVR stage 3. These results showed that when CTGF was injected into the vitreous of a rabbit having a mild degree of PVR, the membranes became densely fibrotic. Therefore, in animal models of mild PVR, intravitreous CTGF addition stimulated and enhanced the development of intraocular fibrosis. These results also showed direct evidence that CTGF induced pathologic fibrosis *in vivo* in an animal model of ocular disease. These results also showed that CTGF induces ocular cell proliferation and acts, at least in part, in the context of other growth factors associated with ocular disorders to induce ocular fibrosis.

**TABLE 4**

| CTGF enhances PDGF-induced PVR in rabbit model | | | | |
|---|---|---|---|---|
| RPE Cell Injected | PDGF Injected | rhCTGF Injected | RPE Stage | Histology |
| 100,000 RPE cells | 50 ng PDGF | No CTGF | Stage 3 | Retinal detachment Thin paucicellular membranes |
| 100,000 RPE cells | 50 ng PDGF | 200 ng CTGF¹ | Stage 5 | Thick, fibrotic membranes |

| | | | | |
|---|---|---|---|---|
| ¹ rhCTGF injected 1 week after RPE cell and PDGF injection | | | | |

### Example 18: Adenovirus-CTGF expression vector construction

Adenovirus expression vectors were designed and constructed for adenovirus-mediated expression of CTGF and fragments of CTGF for use in various *in vivo* and *in vitro* experiments. A CTGF-containing adenovirus expression vector construct (pAd-CTGF) was prepared using the AdEasy system, commercially available from Qbiogene (Montreal, Canada). Components of the system include pAdEasy-1 (the viral backbone, Catalog No. AES1010) and pShuttle-CMV (Catalog No. AES1021). A full-length human CTGF cDNA (DB60R32, GenBank Accession No. M92934, GI:180923) was obtained from Dr. Gary Grotendorst (University of Miami Medical School). (Bradham et al. (1991) J Cell Biol 114:1285-1294.) CTGF cDNA was excised from pBluescript II KS (DB60R32) with the restriction enzymes BamHI (which cleaves pBluescript II KS at position 689) and SwaI (which cleaves CTGF cDNA at a position 1277 bp downstream (i.e., located 3') to the 5' end. The CTGF cDNA fragment was then inserted into pShuttle-CMV, previously digested with the restriction enzymes BgIII and EcoRV, using standard molecular cloning techniques. CTGF expression from the resulting pShuttle-CMV-CTGF construct was confirmed by transient expression of and western blot analysis of expressed proteins, using standard methods.

The pShuttle-CMV-CTGF expression plasmid was used to generate a recombinant adenovirus by bacterial recombinantion with pAdEasy-1 by following the manufacturer's procedures. Briefly, pShuttle-CMV-CTGF was linearized then co-transfected (by electorporation) with pAdEasy-1 into competent *E. coli* BJ-5183 cells. The resulting clones were cultured in LB-KM medium for 16 hr and the recombinant pAd-CTGF plasmid was extracted by alkaline extraction methods. The control plasmids pAd-GFP (containing green fluorescent protein) and pAd-e1E3del.pSCMV (control adenovirus plasmid containing no insert) were also prepared. The recombinant plasmids sequences were confirmed by restriction enzyme mapping using several restriction enzymes. Automated DNA sequencing using an ABI prism 310 automated DNA sequencer further confirmed the CTGF cDNA sequence in pAd-CTGF. Isolated plasmids were transformed to stable *E. coli* JM109 cells. Recombinant plasmids were subsequently linearized with the restriction enzyme Pac1, and transfected with Lipofectamine (GIBCO/BRL, Grand Island, NY) into 293 packaging cells containing E1 and E3 genes. After transfection, the cells were collected and subject to three rounds of freeze-thaw. The supernatants were collected. Viral particles were purified by CsCl gradient ultracentrifuge using 1.30 gravity CsCl solution in 10 mM Tris-HCl, pH 7.4. The titer of the virus was determined by PFU assay.

A replication-deficient recombinant adenovirus gene containing a cDNA fragment encoding human CTGF N-terminal fragments or C-terminal fragments is constructed as follows. Poly(A)⁺ RNA is isolated from cultured human RPE cells using a FastTrack Kit (Invitrogen, San Diego, CA), according to methods described by the manufacturer. First strand cDNA is synthesized at 42°C with M-MLV reverse transcriptase (Gibco/BRL). PCR reaction is performed using oligonucleotide sequence primers specific for human N-terminal CTGF and C-terminal CTGF, and can be selected by standard methods. The use of these primers results in a CTGF-specific DNA amplification product encoding an N-terminal fragment of human CTGF and a C-terminal fragment of CTGF.

PCR amplification is performed using a Perkin-Elmer/Centur DNA thermal cycler, using, fro example, the following conditions: denaturation at 94°C for 1 minute; annealing at 60°C for 1 minute; and extension at 72°C for 45 seconds. Thirty cycles of PCR are performed. The resulting amplification product is isolated and subcloned into the vector pCR 2.1 (Invitrogen Corp.). The DNA sequences of the CTGF N-terminal and C-terminal fragment cDNAs are confirmed by sequencing, using an ABI prism 310-auto sequencer (ABI, USA). The CTGF N-terminal fragment cDNA and C-terminal fragment cDNA is excised from the pCR 2.1 vector construct by digestion using the restriction enzymes Xbal and Xhol. The resulting restriction fragment is isolated and subcloned into the XbaI-XhoI cloning site of pAdTrack-CMV shuttle vector (ADENO-QUEST, Quantum Biolab Inc., Montreal, Canada). pAdTrack-CMV is a shuttle plasmid useful for homologous recombination, and includes the following elements: adenovirus type 5, *E. coli* duplication origin, kanamycin resistance gene, eGFP gene (enhanced green fluorescent protein (Clontech, Palo Alto, CA) whose expression is driven by human cytomegalovirus immediate-early promoter/enhancer, and a multiple cloning site into which DNA is subcloned and expressed from the CMV promoter). The pAdTrack-CMV vector, which includes CTGF N-terminal fragment cDNA or CTGF C-terminal fragment cDNA, is linearized by the restriction enzyme Pme1, and subsequently co-transfected with pAdEasy-1 (ADENO-QUEST, Quantum Biolab Inc.) by electroporation into competent *E. coli* BJ-5183 cells.

The resulting clones were cultured in LB-KM medium for 16 hr and the recombinant plasmids (pAdEasy-CTGF-N, pAdEasy-GFP) were extracted by alkaline extraction methods. The control vector pAdEasy-GFP was constructed using the method described above, excluding insertion of CTGF cDNA. The recombinant plasmid sequences were confirmed by restriction enzyme mapping using several restriction enzymes (e.g., EcoR1, Xho1, Sal1, BamH1, and Pac1). Isolated plasmids were transformed to stable *E. coli* JM109 cells. Recombinant plasmids were subsequently linearized with the restriction enzyme Pac1, and transfected with Lipofectamine (GIBCO/BRL, Grand Island, NY) into 293 packaging cells containing E1 and E3 genes. The sequence of the CTGF cDNA in the vector was confirmed by automated DNA sequencing using an ABI prism 310 auto sequencer. After transfection, the cells were collected, frozen, and thawed three times. The supernatants were collected. Viral particles were purified by CsCl gradient ultracentrifuge using 1.30 gravity CsCl solution in 10 mM Tris-HCl, pH 7.4. The titer of the virus was determined by PFU assay.

### Example 19: Animal model of PVR with pAd-CTGF-infected RPE cells

As shown in the Examples above, the present invention established that RPE cells express and respond to CTGF, and that RPE cells associated with human PVR membranes are positive for CTGF protein expression. These data indicated a role for CTGF in the initiation, formation, development, and maintenance of PVR and PVR membranes associated with various ocular disorders. In the experiments described above in Example 17, RPE cells were injected into the rabbit eye along with exogenously added rhCTGF. The effect observed was relatively minor (See Table 3), possibly due to lack of sustained CTGF exposure or presence within the ocular environment.

In order to investigate the consequence of continual expression and exposure of CTGF within the eye, as would be observed in developing ocular disorders, the rabbit eye model of PVR described above was modified to provide continual CTGF expression within the eye. The modification included the injection of RPE cells previously infected with adenovirus constructs designed to express rhCTGF, as described below.

Confluent cultures of rabbit RPE cells were washed with serum-free growth media and infected with the 10⁷ PFU of a replication-defective adenovirus vector containing CTGF (Ad-CTGF), constructed as described above in Example 18. After 60 minutes, the medium was replaced with fresh medium containing 2% fetal bovine serum. The infected cells were then incubated for 48-72 hrs at 37°C, after which the cells were removed from the plates by trypsin digestion, washed, and resuspended to 1x10⁷ cells/ml in PBS. Using this procedure, greater than 80% of the cultured RPE cells were infected with Ad-CTGF adenovirus.

Three groups of animals were examined in the following study. Negative control animals were injected with RPE cells that had not been infected with adenovirus. The experimental group of animals was injected RPE cells infected with Ad-CTGF. A third group of animals was injected with RPE cells infected with Ad-GFP, which served as an adenovirus control. Additionally, Ad-GFP-infected cells express green fluorescent protein expression, which allowed monitoring of the fate of injected cells using a green fluorescent protein marker. Prior to injection of RPE cells (either adenovirus-infected or control RPE cells) into pigmented rabbit eyes, the intraocular pressure of the rabbit eye was first reduced by withdrawal of 0.2 ml of aqueous humor using a 23-guage needle. With the use of an operating microscope, a 27-gauge needle connected to a tuberculin syringe containing 100,000 adenovirus-infected RPE cells in 0.1 ml PBS was injected into the vitreous by inserting the needle through the sclera, 3 mm posterior to the limbus.

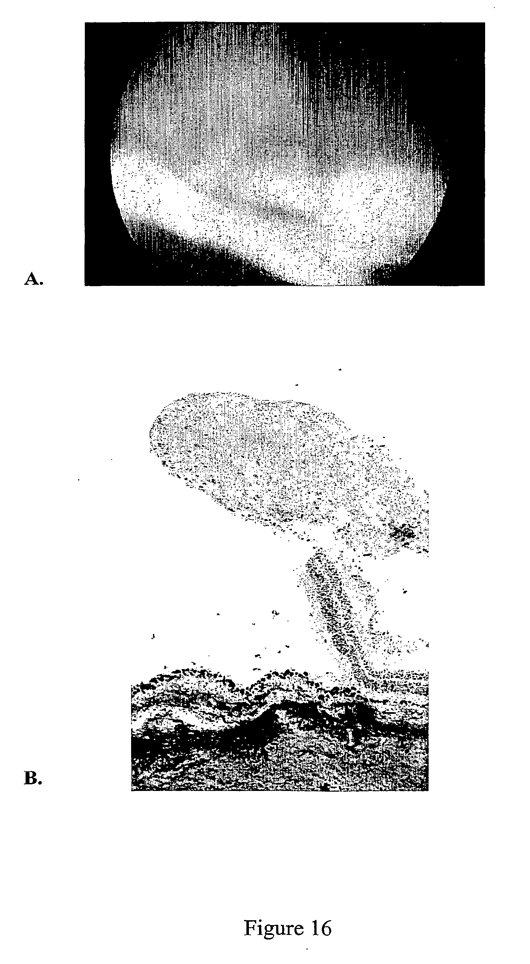

Intravitreous injection of 100,000 RPE cells infected with Ad-CTGF resulted in the formation of a thick membrane, which developed from the optic nerve and extended into the vitreous. (Figures 16A and 16B.) Histological analysis showed the presence of cellular and fibrotic epiretinal membranes. Vitreous haze was evident up to 14 days post-injection; the anterior chamber of the eye remained clear.

PVR developed in eyes injected with Ad-CTGF. No retinal break was observed. Evidence of PVR included the appearance of preretinal membranes, distortion of myelinated fibers and retinal vessels, fixed retinal folds, and traction retinal detachment. Additionally, a mass of fibrotic tissue was present in the eye cup. Histological analysis showed a dense fibrotic epiretinal membrane. Neither PVR nor formation of fibrotic epiretinal membranes developed in saline-inj ected control animals, in animals injected with control adenovirus, or in animals injected with control, non-adenovirus infected RPE cells.

In combination, these results demonstrated that intravitreal injection of RPE cells over-expressing rhCTGF induced ocular fibrosis. Specifically, CTGF addition to an eye in which activated RPE or cellular RPE membranes are present stimulated the formation of intraocular fibrosis, including the transformation of cellular RPE membranes into paucicellular, myofibroblastic, densely fibrotic epiretinal membranes. Additionally, these results indicated that over expression of CTGF initiated the development of PVR without the addition of exogenous growth factors or cytokines typically found in PVR membranes, such as, for example, PDGF, TNFα, TGFβ, HGF, VEGF, IL-1, and IL-6. Therefore, in this animal model of ocular disorder, CTGF provided the initial triggering event leading to fibrosis within a developing non-fribrotic cellular epiretinal membrane. These results showed direct evidence that CTGF induced fibrosis *in vivo* in an animal model of ocular disease.

### Example 20: Subretinal - intravitreous injection of adenovirus model

A pars plana incision is made 3 mm behind the limbus in pigmented rabbits with a stiletto blade. A glass micropipette connected to a micro syringe is inserted under vision control using the operating microscope and contact lens inserted. The retina is gently touched by the micropipette at a distance of 2-3 disc diameters from the optic disc, creating a small retinal hole. Eyes that develope hemorrhage as a result of this procedure are excluded from further study. Two groups of animals are examined in the present study. The experimental group of animals is injected with 50 µl Ad-CTGF. The second group of animals is injected with Ad-GFP, which serves as a control vector and a means to determine which cells were transfected by monitoring expression of green fluorescent protein marker.

### Example 21: Dispase model of PVR

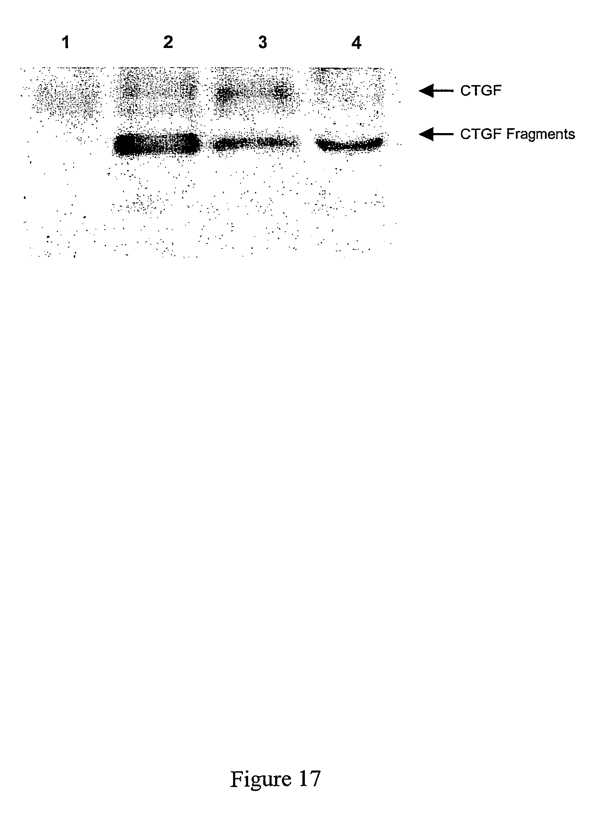

PVR was induced in rabbit eyes using subretinal injection of dispase, using the model and a procedure adapted from that previously described. (Frenzel et al. (1998) Invest Ophthamol Vis Sci 39:2157-2164.) A subretinal bleb was formed as described above using 50 µl (0.05 U) of Dispase (Sigma Chemical Co.) in PBS. Retinal detachment was induced in approximately 75% of the injected rabbits one week after surgery, and in approximately 100% of the injected animals two weeks following surgery. Epiretinal membranes showed moderate fibrosis. Western blot analysis was performed on vitreous aspirates of these animals, as described below in Example 22. As shown in Figure 17, lane 4, the vitreous of these animals demonstrated increased expression of CTGF and fragments of CTGF above that observed in control conditions (Figure 17, lane 1).

### Example 22: Western blot of vitreous aspirates from animal models of PVR

Aspirates of rabbit vitreous from various animal models of PVR were examined for CTGF expression by immunoblot analysis. Assays were performed to determine whether CTGF or CTGF fragments accumulated in the vitreous of rabbits with PVR. Results from these experiments are shown in Figure 17. Positions of CTGF and CTGF fragments are indicated by arrows in Figure 17. Normal rabbit vitreous (Figure 17, lane 1) contained low levels of CTGF. PVR was induced by intravitreous injection of rabbit RPE cells previously infected with Ad-CTGF, as described above in Example 19. In these animals, PVR developed initially after 1 week (Figure 17, lane 3) and became progressively fibrotic after 2 weeks (Figure 17, lane 2). There is predominant expression of CTGF fragments (approximately 18 kDa) in the vitreous of these animals.

### Example 23: The effect of CTGF on the progression of gliosis in a mouse organ culture PVR model

Ocular gliosis is associated with hypertrophy and proliferation of ocular glial cells. Ocular glial cells, including Müller cells and astrocytes, maintain the physical environment and provide structural support to the retina. Ocular gliosis can affect contraction of the retina and can increase the occurrence of retinal detachment.

Eyes from the mouse strain c57B1/6 were used. Eyes were cut at the equator and the cornea and lens removed. The posterior eyecup was placed in 24-well culture plates in Neurobasal medium containing 10% FBS. CTGF (50 ng/ml) was added to the culture medium on the second day of organ culture. Cultures were followed for 7 days for changes including development of retinal folds and rolling up of the retinal edge. Determinations were made by review of photographs and by histology. CTGF stimulated the formation of both retinal folds and rolling of retinal edges when compared with control cultures. Immunohistochemical staining using a glial fibrillary acidic protein (GFAP) antibody revealed that in the areas of pathology, there was a proliferation of GFAP+ glial cells. These results showed that CTGF induced gliosis in perivascular astrocytes. The results obtained with these explant cultures also suggested that CTGF plays an important role in the pathogenesis of PVR by stimulating retinal gliosis and contraction.

## Claims

1. An agent that decreases expression or activity of CTGF or fragments thereof for use in preventing or treating an ocular disorder associated with connective tissue growth factor (CTGF) in a subject, wherein the agent is a) an antibody specifically reactive with CTGF or a fragment or subunit thereof and that neutralizes the biological activity of CTGF or a fragment thereof; or b) an antisense oligonucleotide that inhibits expression and activity of CTGF or fragments thereof; and the ocular disorder is selected from macular degeneration and proliferative vitreoretinopathy.

2. The agent of claim 1 for the use of that claim, wherein the subject is a mammalian subject.

3. The agent of claim 1 for the use of that claim, wherein the subject is a human subject.

4. The agent according to any of the preceding claims for the use of that claim, wherein the agent is to be delivered to the eye.

5. The agent according to any of the preceding claims for the use of that claim, wherein the agent is to be delivered to the ocular surface.

6. The agent according to any of the preceding claims for the use of that claim, wherein the agent is to be delivered in an eye drop formulation.

7. The agent according to any of the preceding claims for the use of that claim, wherein the agent is an antibody.

8. The agent according to any of the preceding claims for the use of that claim, wherein the ocular disorder is macular degeneration.

9. The agent according to any of claims 1-7 for the use of that claim, wherein the ocular disorder is proliferative vitreoretinopathy.

10. The agent according to any of the preceding claims for the use of that claim, wherein the macular degeneration is selected from age-related macular degeneration, the dry form of macular degeneration and the wet form of macular degeneration.

11. The agent according to any of the preceding claims for the use of that claim, wherein the medicament is for treating or preventing choroidal neovascularization associated with the macular degeneration.

12. The agent according to claim 9 for the use of that claim, wherein the proliferative vitreoretinopathy is associated with retinal detachment.

13. Use of an agent that decreases expression or activity of CTGF or fragments thereof in the manufacture of a medicament for preventing or treating an ocular disorder associated with connective tissue growth factor (CTGF) in a subject, wherein the agent is a) an antibody specifically reactive with CTGF or a fragment or subunit thereof and that neutralizes the biological activity of CTGF or a fragment thereof; or b) an antisense oligonucleotide that inhibits expression and activity of CTGF or fragments thereof; and the ocular disorder is selected from macular degeneration and proliferative vitreoretinopathy.

## Patentansprüche

1. Mittel, welches die Expression oder Aktivität von CTGF oder Fragmenten davon senkt, zur Verwendung bei der Vorbeugung oder Behandlung einer okularen bzw. Augenstörung, die mit dem Bindegewebswachstumsfaktor (CTGF) in Verbindung steht, bei einem Individuum, wobei das Mittel a) ein Antikörper ist, der spezifisch reaktiv ist mit CTGF oder einem Fragment oder einer Untereinheit davon, und welcher die biologische Aktivität von CTGF oder einem Fragment davon neutralisiert; oder b) ein Antisinn-Oligonukleotid ist, welches die Expression und Aktivität von CTGF oder Fragmenten davon inhibiert; und wobei die Augenstörung aus Makuladegeneration und proliferativer Vitreoretinopathie gewählt ist.

2. Mittel von Anspruch 1 zur Verwendung von dem Anspruch, wobei das Individuum ein Säuger-Individuum ist.

3. Mittel von Anspruch 1 zur Verwendung von dem Anspruch, wobei das Individuum ein menschliches Individuum ist.

4. Mittel gemäß einem der vorhergehenden Ansprüche für die Verwendung von dem Anspruch, wobei das Mittel an das Auge zu verabreichen ist.

5. Mittel gemäß einem der vorhergehenden Ansprüche für die Verwendung von dem Anspruch, wobei das Mittel an die Augenoberfläche zu verabreichen ist.

6. Mittel gemäß einem der vorhergehenden Ansprüche für die Verwendung von dem Anspruch, wobei das Mittel in einer Augentropfenformulierung zu verabreichen ist.

7. Mittel gemäß einem der vorhergehenden Ansprüche für die Verwendung von dem Anspruch, wobei das Mittel ein Antikörper ist.

8. Mittel gemäß einem der vorhergehenden Ansprüche für die Verwendung von dem Anspruch, wobei die Augenstörung Makuladegeneration ist.

9. Mittel gemäß einem der Ansprüche 1 bis 7 für die Verwendung von dem Anspruch, wobei die Augenstörung proliferative Vitreoretinopathie ist.

10. Mittel gemäß einem der vorhergehenden Ansprüche für die Verwendung von dem Anspruch, wobei die Makuladegeneration aus mit dem Alter im Zusammenhang stehender Makuladegeneration, der trockenen Form der Makuladegeneration und der nassen Form der Makuladegeneration gewählt ist.

11. Mittel gemäß einem der vorhergehenden Ansprüche für die Verwendung von dem Anspruch, wobei das Medikament zur Behandlung oder Vorbeugung von choroidaler Neovaskularisierung, die mit der Makuladegeneration im Zusammenhang steht, ist.

12. Mittel gemäß Anspruch 9 für die Verwendung von dem Anspruch, wobei proliferative Vitreoretinopathie mit einer Netzhautablösung im Zusammenhang steht.

13. Verwendung eines Mittels, welches die Expression oder Aktivität von CTGF oder Fragmenten davon senkt, bei der Herstellung eines Medikaments zur Vorbeugung oder Behandlung einer Augenstörung, die mit dem Bindegewebswachstumsfaktor (CTGF) in Verbindung steht, bei einem Individuum, wobei das Mittel a) ein Antikörper ist, der spezifisch reaktiv ist mit CTGF oder einem Fragment oder einer Untereinheit davon, und welcher die biologische Aktivität von CTGF oder einem Fragment davon neutralisiert; oder b) ein Antisinn-Oligonukleotid ist, welches die Expression und Aktivität von CTGF oder Fragmenten davon inhibiert; und wobei die Augenstörung aus Makuladegeneration und proliferativer Vitreoretinopathie gewählt ist.

## Revendications

1. Agent qui diminue l'expression ou l'activité du CTGF ou de fragments de celui-ci destiné à être utilisé dans la prévention ou le traitement d'un trouble oculaire associé au facteur de croissance de tissu conjonctif (CTGF) chez un sujet, l'agent étant a) un anticorps spécifiquement réactif avec le CTGF ou un fragment ou une sous-unité de celui-ci et qui neutralise l'activité biologique du CTGF ou d'un fragment de celui-ci ; ou b) un oligonucléotide antisens qui inhibe l'expression et l'activité du CTGF ou de fragments de celui-ci; et le trouble oculaire étant choisi parmi la dégénérescence maculaire et la vitréoréthinopathie proliférante.

2. Agent selon la revendication 1 pour l'utilisation de cette revendication, le sujet étant un sujet mammifère.

3. Agent selon la revendication 1 pour l'utilisation de cette revendication, le sujet étant un sujet humain.

4. Agent selon l'une quelconque des revendications précédentes pour l'utilisation de cette revendication, l'agent devant être administré à l'oeil.

5. Agent selon l'une quelconque des revendications précédentes pour l'utilisation de cette revendication, l'agent devant être administré à la surface oculaire.

6. Agent selon l'une quelconque des revendications précédentes pour l'utilisation de cette revendication, l'agent devant être administré dans une formulation de gouttes ophtalmiques.

7. Agent selon l'une quelconque des revendications précédentes pour l'utilisation de cette revendication, l'agent étant un anticorps.

8. Agent selon l'une quelconque des revendications précédentes pour l'utilisation de cette revendication, le trouble oculaire étant la dégénérescence maculaire.

9. Agent selon l'une quelconque des revendications 1-7 pour l'utilisation de cette revendication, le trouble oculaire étant la vitréoréthinopathie proliférante.

10. Agent selon l'une quelconque des revendications précédentes pour l'utilisation de cette revendication, la dégénérescence maculaire étant choisie parmi la dégénérescence maculaire liée à l'âge, la forme sèche de la dégénérescence maculaire et la forme humide de la dégénérescence maculaire.

11. Agent selon l'une quelconque des revendications précédentes pour l'utilisation de cette revendication, le médicament étant destiné au traitement ou à la prévention d'une néovascularisation choroïdienne associée à la dégénérescence maculaire.

12. Agent selon la revendication 9 pour l'utilisation de cette revendication, la vitréoréthinopathie proliférant étant associée à un décollement de la rétine.

13. Utilisation d'un agent qui diminue l'expression ou l'activité du CTGF ou de fragments de celui-ci dans la fabrication d'un médicament pour la prévention ou le traitement d'un trouble oculaire associé au facteur de croissance de tissu conjonctif (CTGF) chez un sujet, l'agent étant a) un anticorps spécifiquement réactif avec le CTGF ou un fragment ou une sous-unité de celui-ci et qui neutralise l'activité biologique du CTGF ou d'un fragment de celui-ci ; ou b) un oligonucléotide antisens qui inhibe l'expression et l'activité du CTGF ou de fragments de celui-ci ; et le trouble oculaire étant choisi parmi la dégénérescence maculaire et la vitréoréthinopathie proliférante.


### SEQUENCE LISTING

<110> Hinton, David R.
   He, Shikun
   oliver, Noelynn A.
<120> METHODS FOR INHIBITING OCULAR PROCESSES
<130> FP0813 US
<150> US 60/339,547
   <151> 2001-12-11
<160> 8
<170> PatentIn version 3.2
<210> 1
   <211> 20
   <212> DNA
   <213> Homo sapiens
<400> 1
   gcatccgtac tcccaaaatc 20
<210> 2
   <211> 20
   <212> DNA
   <213> Homo sapiens
<400> 2
   cttcttcatg acctcgccgt 20
<210> 3
   <211> 22
   <212> DNA
   <213> Bos taurus
<400> 3
   gatcatagga ctgtattagt gc 22
<210> 4
   <211> 20
   <212> DNA
   <213> Bos taurus
<400> 4
   ctgacttaga gacgaacttg 20
<210> 5
   <211> 33
   <212> DNA
   <213> Homo sapiens
<400> 5
   gctccgcccg cagtgggatc catgaccgcc gcc 33
<210> 6
   <211> 30
   <212> DNA
   <213> Homo sapiens
<400> 6
   ggatccggat cctcatgcca tgtctccgta 30
<210> 7
   <211> 22
   <212> DNA
   <213> Rattus norvegicus
<400> 7
   ctccagtctg cagaaggtat tc 22
<210> 8
   <211> 20
   <212> DNA
   <213> Rattus norvegicus
<400> 8
   caaccgcaag attggagtgt 20